# EUROPEAN PATENT SPECIFICATION

(11) **EP 0 561 639 B1**
(45) Date of publication and mention of the grant of the patent: **15.05.2002**
(21) Application number: 93302064.6
(22) Date of filing: 18.03.1993
(51) Int. Cl.: C07K 7/56, A61K 38/12

(54) **Cyclic peptide antifungal agents and process for preparation thereof**
Zyklische Peptide mit antifungischer Wirkung und Verfahren zu ihrer Herstellung
Dérivés cyclopeptidiques antifongiques et procédé de leur préparation

(30) Priority: 19.03.1992 US 854117; 16.12.1992 US 992390
(43) Date of publication of application: 22.09.1993
(73) Proprietor: ELI LILLY AND COMPANY, Indianapolis, Indiana 46285 (US)
(72) Inventor: Burkhardt, Frederick Joseph, Indianapolis, Indiana 46236 (US); Debono, Manuel, Indianapolis, Indiana 46208 (US); Nissen, Jeffrey Scott, Indianapolis, Indiana 46217 (US); Turner Jr., William Wilson, Bloomington, Indiana 47401 (US)
(74) Representative: Goldin, Douglas Michael

(56) References cited:
- EP-A- 0 447 186
- EP-A- 0 448 343
- EP-A- 0 448 354

## Description

### Background of the Invention

This invention relates to cyclic peptide antifungal agents. In particular, it relates to acyl derivatives of the echinocandin class of cyclic peptide antifungal agents; to methods for treating antifungal and parasitic infections, and to formulations useful in the methods.

The compounds provided by this invention are semi-synthetic antifungal agents in that they are derived from the cyclic peptide antifungals which are produced by culturing various microorganisms. A number of cyclic peptide antifungals are known. Among these are echinocandin B (A30912A), aculeacin, mulundocandin, sporiofungin, L-671,329, FR901379, and S31794/F1. All such antifungals are structurally characterized by a cyclic hexapeptide core, or nucleus, the amino group of one of the cyclic amino acids bearing a fatty acid acyl group forming a side chain off the core or nucleus. For example, echinocandin B has a linoleoyl side chain while aculeacin has a palmitoyl side chain. These fatty acid side chains of the cyclic hexa- peptides can be removed by enzymatic deacylation to provide the free nucleus. (Formula (1), hereinafter, wherein R₂ is hydrogen.) Reacylation of the amino group of the nucleus provides semisynthetic antifungal compounds. For example, the echinocandin B nucleus provides a number of antifungal agents when reacylated with certain unnatural side chain moieties (see *Debono*, U.S. Pat. No. 4,293,489). Among such antifungal compounds is cilofungin which is represented by the formula (1) wherein R is methyl, R₁ is hydrogen and R₂ is p-(n-octyloxy)benzoyl.

Enzymatic deacylation of the cyclic hexapeptides is carried out with deacylase produced by the organism Actinoplanes utahensis and related microorganisms as described by *Abbott et al*., U.S. Pat. No. 4,293,482.

The present invention provides acylated cyclic hexapeptides having unique side chain acyl groups which, inter alia impart enhanced antifungal and antiparasitic potency e.g. against pathogenic strains of Candida albicans. Also provided is a process for removing the aminal and benzylic hydroxy groups to result in a dideoxy compound of formula (1) (R = H).

### Summary of the Invention

The compounds provided by this invention are represented by the following formula (1): 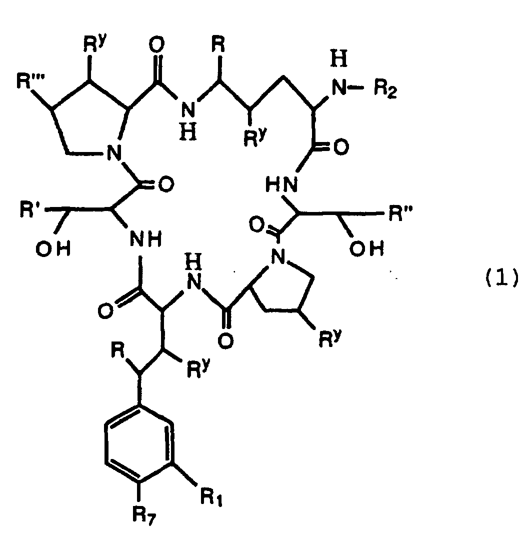 wherein
R' is hydrogen, methyl or NH₂C(O)CH₂-;
R" and R"' are independently methyl or hydrogen;
R and R^{y} are independently hydroxy or hydrogen;
R₁ is hydroxy, hydrogen or hydroxysulfonyloxy;
R₇ is hydroxy, hydrogen, hydroxysulfonyloxy or phosphonooxy; and

I) R₂ is a substituted benzoyl group represented by the formula 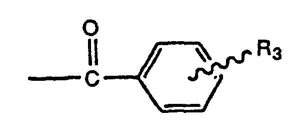 wherein
   R₃ is quinolyl; or
II) R₂ is an acyl group represented by the formula 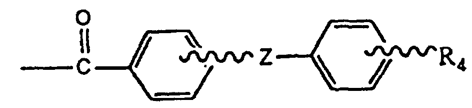 wherein
   Z is -C≡C-, -CH=CH-, or a carbon to carbon bond;

   A) R₄ is C₃-C₁₂ cycloalkyl, C₇-C₁₀ bicycloalkyl, C₇-C₁₄ tricycloalkyl, C₃-C₁₂ cycloalkoxy, naphthyl, pyridyl, thienyl, benzothienyl, quinolyl or phenyl; or
   B) R₄ is phenyl substituted by amino, C₁-C₁₂ alkylthio, halogen, C₁-C₁₂ alkyl, C₂-C₁₂ alkenyl, C₂-C₁₂ alkynyl, C₁-C₁₂ substituted alkyl, C₂-C₁₂ substituted alkenyl, C₂-C₁₂ substituted alkynyl, C₁-C₁₂ alkoxy, trifluoromethyl, phenyl, substituted phenyl, phenyl substituted with a polyoxa-alkyl group represented by the formula

      -O-(CH₂)ₘ-[O-(CH₂)ₙ]ₚ-O-(C₁-C₁₂ alkyl)

      wherein m and n are integers of from 2 to 4, and p is 0 or 1; or
   C) R₄ is phenyl substituted with C₁-C₆ alkoxy substituted by fluoro, bromo, chloro or iodo; or
   D) R₄ is a group represented by the formula

      -Y-R₆
   wherein
   Y is -C≡C- or -C=C-; and
   R₆ is C₁-C₁₂ alkyl, C₁-C₁₂ substituted alkyl; C₃-C₁₂ cycloalkyl, C₇-C₁₀ bicycloalkyl, C₇-C₁₄ tricycloalkyl, phenyl, C₃-C₁₂ cycloalkenyl, naphthyl, benzothiazolyl, thienyl, phenyl substituted by amino, C₁-C₁₂ alkylthio, halogen, C₁-C₁₂ alkyl, C₂-C₁₂ alkenyl, C₂-C₁₂ alkynyl, C₁-C₁₂ alkoxy, trifluoromethyl, -O-(CH₂)_{p'}-W-R₅ wherein p' is an integer of from 2 to 4; W is pyrrolidino, piperidino or piperazino, and R₅ is hydrogen, C₁-C₁₂ alkyl, C₃-C₁₂ cycloalkyl, benzyl or C₃-C₁₂ cycloalkylmethyl; or C₁-C₆ alkoxy substituted by fluoro, bromo, iodo or chloro; or
   R₆ is a phenyl substituted by a polyoxa-alkyl group represented by the formula

      -O-(CH₂)ₘ-[O-(CH₂)ₙ]ₚ-O-(C₁-C₁₂ alkyl)

      wherein m, n and p are as defined above; or
   R₂ is an acyl group represented by the formula 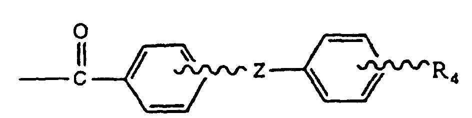 wherein
      Z is -C≡C- or -CH=CH-;

   A) R₄ is hydrogen, C₂-C₁₂ alkynyl, C₂-C₁₂ substituted alkynyl, C₁-C₁₂ alkoxy; or
   B) R₄ is C₁-C₁₂ alkoxy substituted with C₃-C₁₂ cycloalkyl, C₇-C₁₀ bicycloalkyl, C₇-C₁₄ tricycloalkyl, C₂-C₁₂ alkynyl, amino, C₁-C₄ alkylamino, di-(C₁-C₄ alkyl)amino, C₁-C₁₂ alkanoylamino, phenyl substituted with a polyoxa-alkyl group represented by the formula

      -O-(CH₂)ₘ-[O-(CH₂)ₙ]ₚ-O-(C₁-C₁₂ alkyl)

      wherein m,n and p are as defined; or
   C) R₄ is C₁-C₁₂ alkoxy substituted with a group of the formula 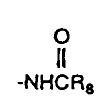 wherein R₈ is C₁-C₆ alkoxy optionally substituted with phenyl; or
   D) R₄ is a group represented by the formula

      -O-(CH₂)_{p'}-W-R₅
   wherein p', W and R₅ are as defined; or
IV) R₂ is a group having the formula 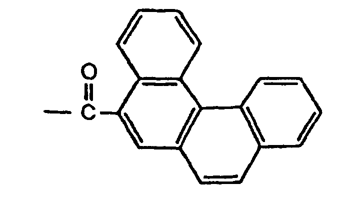 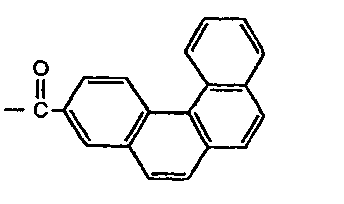 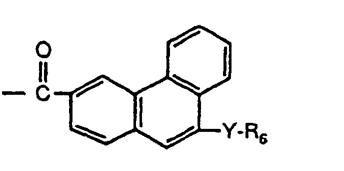 wherein Y and R₆ are as defined above; or
V) R₂ is naphthoyl substituted with R₄
wherein
A) R₄ is C₃-C₁₂ cycloalkyl, C₇-C₁₀ bicycloalkyl, C₇-C₁₄ tricycloalkyl, C₃-C₁₂ cycloalkoxy, naphthyl, pyridyl, thienyl, benzothienyl, quinolyl or phenyl; or
B) R₄ is phenyl substituted by amino, C₁-C₁₂ alkylthio, halogen, C₁-C₁₂ alkyl, C₂-C₁₂ alkenyl, C₂-C₁₂ alkynyl, C₁-C₁₂ substituted alkyl, C₂-C₁₂ substituted alkenyl, C₂-C₁₂ substituted alkynyl, C₁-C₁₂ alkoxy, trifluoromethyl, phenyl, substituted phenyl, phenyl substituted with a polyoxa-alkyl group represented by the formula

   -O-(CH₂)ₘ-[O-(CH₂)ₙ]ₚ-O-(C₁-C₁₂ alkyl)

   wherein m, n and p are as defined; or
C) R₄ is phenyl substituted with C₁-C₆ alkoxy substituted by fluoro, bromo, chloro or iodo; or
D) R₄ is a group represented by the formula

   -Y-R₆

   wherein
   Y has the same meanings as defined above; and
   R₆ is C₁-C₁₂ alkyl, C₁-C₁₂ substituted alkyl; C₃-C₁₂ cycloalkyl, C₇-C₁₀ bicycloalkyl, C₇-C₁₄ tricycloalkyl, phenyl, C₃-C₁₂ cycloalkenyl, naphthyl, benzothiazolyl, thienyl, phenyl substituted by amino, C₁-C₁₂ alkythio, halogen, C₁-C₁₂ alkyl, C₂-C₁₂ alkenyl, C₂-C₁₂ alkynyl, C₁-C₁₂ alkoxy, trifluoromethyl, -O-(CH₂)_{p'}-W-R₅, or C₁-C₆ alkoxy substituted by fluoro, bromo, iodo or chloro; or
   R₆ is a phenyl substituted by a polyoxa-alkyl group represented by the formula

      -O-(CH₂)ₘ-[O-(CH₂)ₙ]ₚ-O-(C₁-C₁₂ alkyl)
   wherein m, n and p are as defined above; and the pharmaceutically acceptable non-toxic salts thereof.

Also provided are formulations and methods for inhibiting parasitic and fungal activity which employ the compounds of the invention, and a process for preparing the dideoxy form of the compounds.

### Detailed Description

The term: "C₁-C₁₂ alkyl" refers to the straight or branched chain alkyl hydrocarbon groups such as, for example, methyl, ethyl, n-propyl, isopropyl, n-butyl, sec-butyl, t-butyl, pentyl, hexyl, heptyl, octyl, nonyl, decyl, undecyl and dodecyl groups; and the like.

The term "C₂-C₁₂ alkenyl" refers to groups such as vinyl, 1-propene-2-yl, 1-butene-4-yl, 1-pentene-5-yl, 1-butene-1-yl, and the like.

The term "C₂-C₁₂ alkynyl" refers to such groups as ethynyl, propynyl, pentynyl, butynyl and the like.

The term "C₁-C₁₂ alkylthio" refers to such groups as methylthio, ethylthio, t-butylthio, and the like.

The term "C₁-C₁₂ alkoxy" refers to the straight or branched chain oxyalkyl groups such as, e.g. methoxy, ethoxy, propoxy, butoxy, heptoxy, octyloxy, dodecyloxy, and the like.

The term C₃-C₁₂ cycloalkoxy" refers to such groups as cyclopropoxy, cyclobutoxy and the like.

The term "C₃-C₁₂ cycloalkenyl" refers to such groups as cyclopropenyl, cyclobutenyl, cyclopentenyl, and the like.

The term "C₁-C₁₂ substituted alkyl," "C₂-C₁₂ substituted alkenyl", and "C₂-C₁₂ substituted alkynyl", denotes the above substituted one or two times with halogen, hydroxy, protected hydroxy, amino, protected amino, C₁-C₇ acyloxy, nitro, carboxy, protected carboxy, carbamoyl, carbamoyloxy, cyano, methylsulfonylamino, phenyl, substituted phenyl, or C₁-C₁₂ alkoxy.

The term "substituted phenyl" is represented by a phenyl group substituted with one, two, or three moieties chosen from halogen, hydroxy, protected hydroxy, cyano, nitro, C₁-C₁₂ alkyl, C₁-C₁₂ alkoxy, carboxy, protected carboxy, carboxymethyl, hydroxymethoyl, amino, aminomethyl trifluoromethyl or N-(methylsulfonylamino)

The term "C₃-C₁₂ cycloalkyl" refers to such groups as cyclopropyl, cyclobutyl, cyclopentyl, cyclohexyl and cycloheptyl.

The term "C₁-C₄ alkylamino" refers to such groups as methylamino, ethylamino, n-butylamino and the like.

The term "di-(C₁-C₄ alkyl)amino" refers to such groups as dimethylamino, diethylamino, di-n-propylamino, di-n-butylamino, methylethylamino, methyl-n-butylamino, and like tertiary amino groups.

The term "C₁-C₁₂ alkanoylamino" refers to such groups as acylamino groups derived from the C₁-C₁₂ carboxylic acids and are exemplified by formamido, acetylamino, propionylamino, butyrylamino, and the like,

The term "C₃-C₁₂ cycloalkylmethyl" refers to those C₃-C₇ cycloalkyls described above further substituted by methyl.

The terms "C₇-C₁₀ bicycloalkyl" and "C₇-C₁₄ tricycloalkyl" refer to such groups as bicyclo[2.2.1.]hept-2-yl, bicyclo[2.2.1.]hep-4-en-2-yl, bicyclo[3.3.1.]nona-3-yl, bicyclo[3.3.1.]nona-2-yl, bicyclo[3.2.1.]oct-2-yl, bicyclo[2.2.2.]oct-2-yl, bicyclo[2.2.2]oct-5-en-2-yl, adamantyl and the like.

The term "dideoxy" refers to compounds of the formula (1) wherein R=H.

The term "inhibiting", such as used in relation to the methods for inhibiting parasitic and fungal activity, is defined to mean its normal definition, i.e., to stop, retard or prophylactically hinder or prevent.

The term "activity", as used in relation to parasitic and fungal activity, includes growth thereof and attending characteristics and results from the existence of the parasite or fungus.

The term "contacting", as used in relation to the methods for inhibiting parasitic and fungal activity by contacting a compound of the invention with a parasite or fungus, is defined to mean its normal definition. However, the term does not imply any further limitations to the process, such as by mechanism of inhibition, and the methods are defined to encompass the spirit of the invention, which is to inhibit parasitic and fungal activity by the action of the compounds and their inherent anti-parasitic and anti-fungal properties, or in other words, the compounds, used in the method are the causative agent for such inhibition.

Examples of acyl groups wherein R₂ is a group represented by the formula 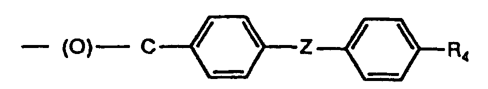 are diphenyl acetylenes (Z=-C≡C-), stilbenes (Z=-CH=CH-), and biphenyls (Z = a carbon to carbon bond). Among examples of such biphenyl groups, wherein Z is a carbon to carbon bond i.e. a phenyl to phenyl bond, are 4-[4-(butyloxy)phenyl]benzoyl, 4-[4-(cyclobutylmethoxy)-phenyl]benzoyl, 4-[4-cyclopentylmethoxy)phenyl]benzoyl, 4-[4-(cyclohexylethoxy)-phenyl]benzoyl, 4-[4-(n-hexyloxy)-phenyl]benzoyl, 4-phenylbenzoyl, 4-[4-(11-amino-undecyloxy)-phenyl]benzoyl, 4-[4-(11-formamidoundecyloxy)phenyl]benzoyl, 4-[4-(isopentyloxy)phenyl]benzoyl, and the like. Examples of diphenylacetylene and stilbene acyl groups, R₂, wherein Z is an acetylenic bond or an ethylene bond are 4-styrylbenzoyl, 4-(4-methoxystyryl)benzoyl, 4-(4-butyloxystyryl)benzoyl, 4-(phenylethynyl)benzoyl, 4-(4-ethoxyphenylethynyl)benzoyl, 4- (4-cyclohexyloxyphenylethynyl)benzoyl, and the like.

Examples of such acyl groups wherein R₄ is represented by the formula -Y-R₆ include 4-[4-(phenylethynyl)phenyl]benzoyl, 4-[4-(phenylethynyl)-phenoxy]benzoyl, 4-[4-(hexynyl)phenyl]benzoyl, 4-[4-(styryl)phenoxy]benzoyl, 4-[4-(4-benzylphenylethynyl)-phenyl]benzoyl, 4-[4-[4-4-methylpiperidino)ethoxy]phenylethynyl]phenyl]benzoyl and like acyl groups. Such acyl groups wherein R₄ is represented by the formula -O-(CH₂)_{p'}-W-R₅ form salts of the basic amino groups of the piperidine and piperazine heterocyclic groups with both organic and inorganic acids such as hydrochloric acid, hydrobromic acid, sulfuric acid and phosphoric acid and with organic acids such as the sulfonic acids, benzenesulfonic acid, toluenesulfonic acid, methanesulfonic acid, acetic acid, chloroacetic acid, trifluoroacetic acid, benzoic acid, isophthalic acid, salicylic acid, citric acid, malic acid, succinic acid, malonic acid and like acids.

The following tables contain further examples of the cyclic peptides represented by the formula (1). Table 1 contains examples of cyclic peptides wherein the acyl group R₂ is of the formula

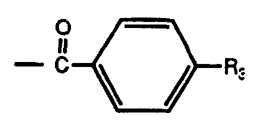

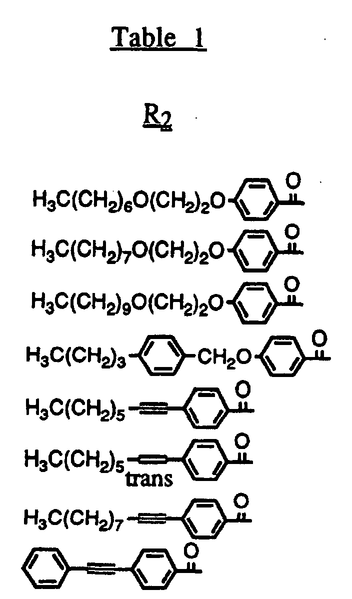

The following Table 2 illustrates the compound of the formula (1) wherein R₂ is represented by the formula

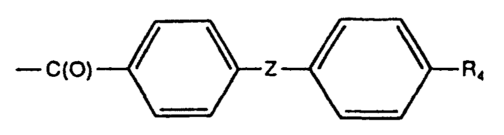

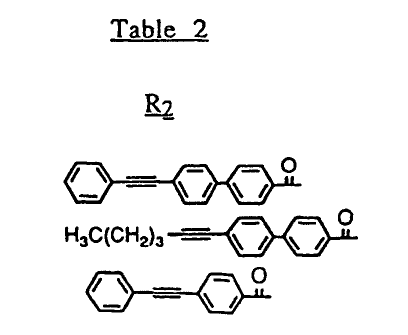

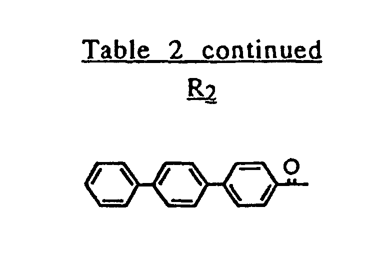

The acyl cyclohexapeptides represented by formula (1) exhibit antiparasitic activity, for example, they are especially active against the infectious fungi Candida albicans and Candida parapsilosis. They also exhibit significant activity against Aspergillus fumigatus. They are active both in vitro and in vivo and accordingly are useful in combating systemic fungal infections.

The compounds of the invention also inhibit the growth of certain organisms primarily responsible for opportunistic infections in immunosuppressed individuals. For example the compounds of the invention inhibit the growth of Pneumocystis carinii the causative organism of pneumocystis pneumonia in AIDS sufferers.

The antifungal activity of the compounds of the invention is determined in vitro in standard agar dilution tests and disc-diffusion tests wherein minimum inhibitory concentrations of the test compounds obtained. Standard in vivo tests in mice are used to determine the effective dose of the test compounds in controlling systemic fungal infections.

Tables 4A-E below contain the minimum inhibitory concentrations (MIC) in micrograms per milliliter (mcg/ml) for compounds of the invention against Candida albicans and Candida parapsilosis, and for certain compounds, the effective dose, ED₅₀, in mice.

In Tables 4A-E, R'=CH₃, R"=CH₃, R'''=CH₃, R^{y}=OH, R₇=OH and R₁=H, In Tables 4A-D, R=OH, while in Table E, R=H.

In Table 4C, R₂ is of the formula 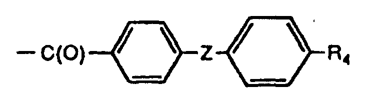 where Z is a carbon-carbon bond and R₄ is as indicated.

**TABLE 4C**

| R₄ | MIC (mcg/ml) | | ED₅₀ (mg/ml) |
|---|---|---|---|
| | C.alb. | C.parap. | |
| -C≡C-C₄H₉ | 0.039 | 2.5 | 1.20 |
| -C≡C-C₆H₅ | 0.039 | 0.625 | 0.60 |
| -C₆H₅ | 0.078 | 10 | 1.3 |

The non-dideoxy compounds of the invention (formula (1) are prepared with the amino nuclei of the cyclic hexapeptides which are represented by the formula when R₂ is hydrogen. These amino nuclei are obtained from the known natural products by the known enzymatic deacylation by which the fatty acid side chains of the natural compounds are removed. For example, echinocandin B which can be represented by the formula (1) wherein R'=R''=R'''= methyl, R is OH, R^{y} is hydroxy, R₁ is H, R₇ is OH, and R₂ is linoleoyl, is deacylated to provide the echinocandin B nucleus (R₂=H) with the deacylase produced by the organism Actinoplanes utahensis as described by U.S. Patent Nos. 4,293,482 and 4,304,716.

The known natural cyclic hexapeptides which are N-deacylated to provide the amino nuclei starting materials include echinocandin B (also known as A-30912A), aculeacin (palmitoyl side chain), tetrahydoechinocandin B (stearoyl side chain), mulundocandin (branched C₁₅ side chain), L-671,329 (C₁₆ branched side chain), S 31794/F1 (tetradecanoyl side chain), sporiofungin (C₁₅ branched side chain) and FR901379 (palmitoyl side chain). The amino nuclei obtained by the N-deacylation are then acylated by employing known amino acylation procedures to provide the N-acyl cyclic hexapeptides represented by the formula (1) wherein R₂ represents the acyl groups defined hereinabove. The acylating moiety is preferably an active ester of the carboxylic acid RCOOH such as the 2,4,5-trichlorophenyl ester. The R₂COOH precursor acids are prepared by the hydrolysis of the nitrile R₂CN or the ester R₂COOC₁-C₄ alk. These nitrile and ester intermediates are prepared by known methods.

The alkoxy aromatic (ie. phenyl and biphenyl) compounds of Tables 9-10 are prepared by one of the two following procedures:
A. The hydroxyaromatic compound (1 equivalent) is dissolved in acetonitrile (200-300 ml) and a base, such as potassium t-butoxide or potassium carbonate,(1-equivalent), is added. An alkyl bromide, iodide, or p-toluenesulfonate (1 equivalent) is then added and the solution is refluxed for 6 hours. The solvent is evaporated in vacuo and the residue is dissolved in ether and 2N sodium hydroxide. The ether layer is dried over magnesium sulfate and evaporated to give the alkoxyaromatic product.
B. The hydroxyaromatic compound (1 equivalent), alkyl alcohol (1 equivalent), and triphenylphosphine (1 equivalent) are dissolved in tetrahydrofuran (200-300 ml) and diethylazodicarboxylate (1 equivalent) is added dropwise over 10 minutes at room temperature. After 17 hours the solvent is removed in vacuo and the residue is dissolved in ether. This organic layer is extracted with 2N sodium hydroxide solution, dried over magnesium sulfate, and evaporated to give a product which is crystallized from ether/pentane or, if the product contains a tertiary amine, the hydrochloride salt is formed and crystallized from methanol/ethyl acetate.

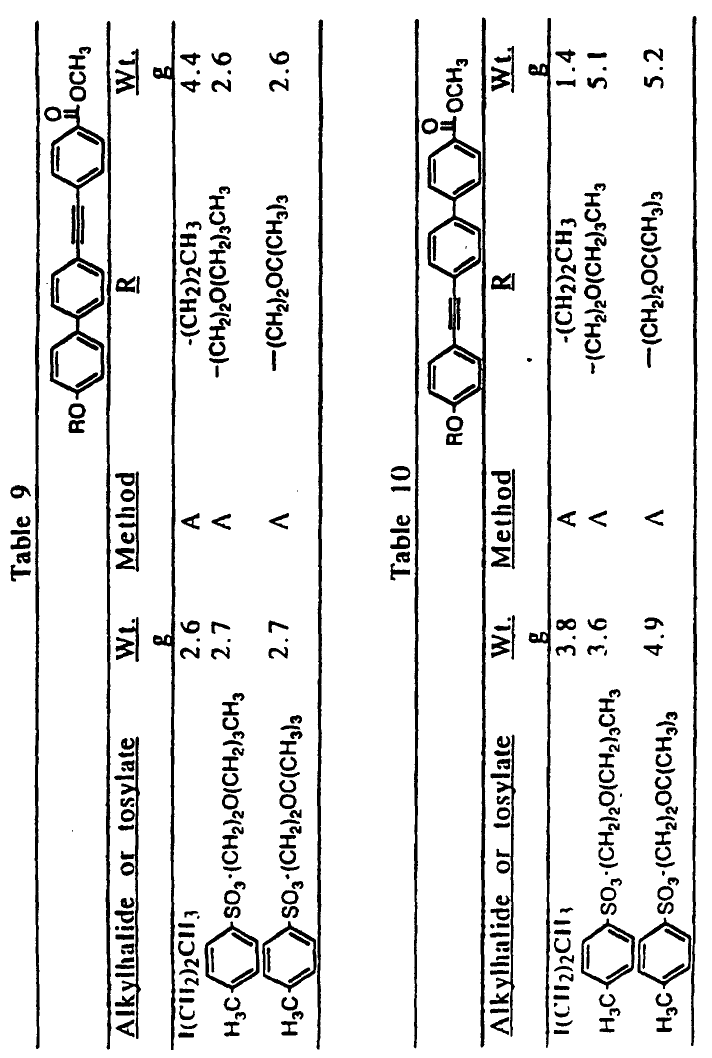

The alkynyl and alkenyl aromatic compounds contained in Tables 11-14 are prepared by the following procedure:

An aromatic bromide, iodide, or trifluoromethane-sulfonate (1 equivalent) is dissolved in acetonitrile (600 ml/0.1 mole of aromatic reactant) under a nitrogen atmosphere. An alkyne or alkene (1 equivalent), triethylamine (2 equivalents), palladium dichloride (0.05 equivalents), triphenylphosphine (0.1 equivalents), and cuprous iodide (0.025 equivalents) are added and the solution is refluxed for 17 hours. The solvent is removed in vacuo and the residue is slurried in ether (300 ml). Solids are removed by filtration and the filtrate is washed with 1N hydrochloric acid solution. The organic layer is dried over magnesium sulfate and evaporated to yield the product.

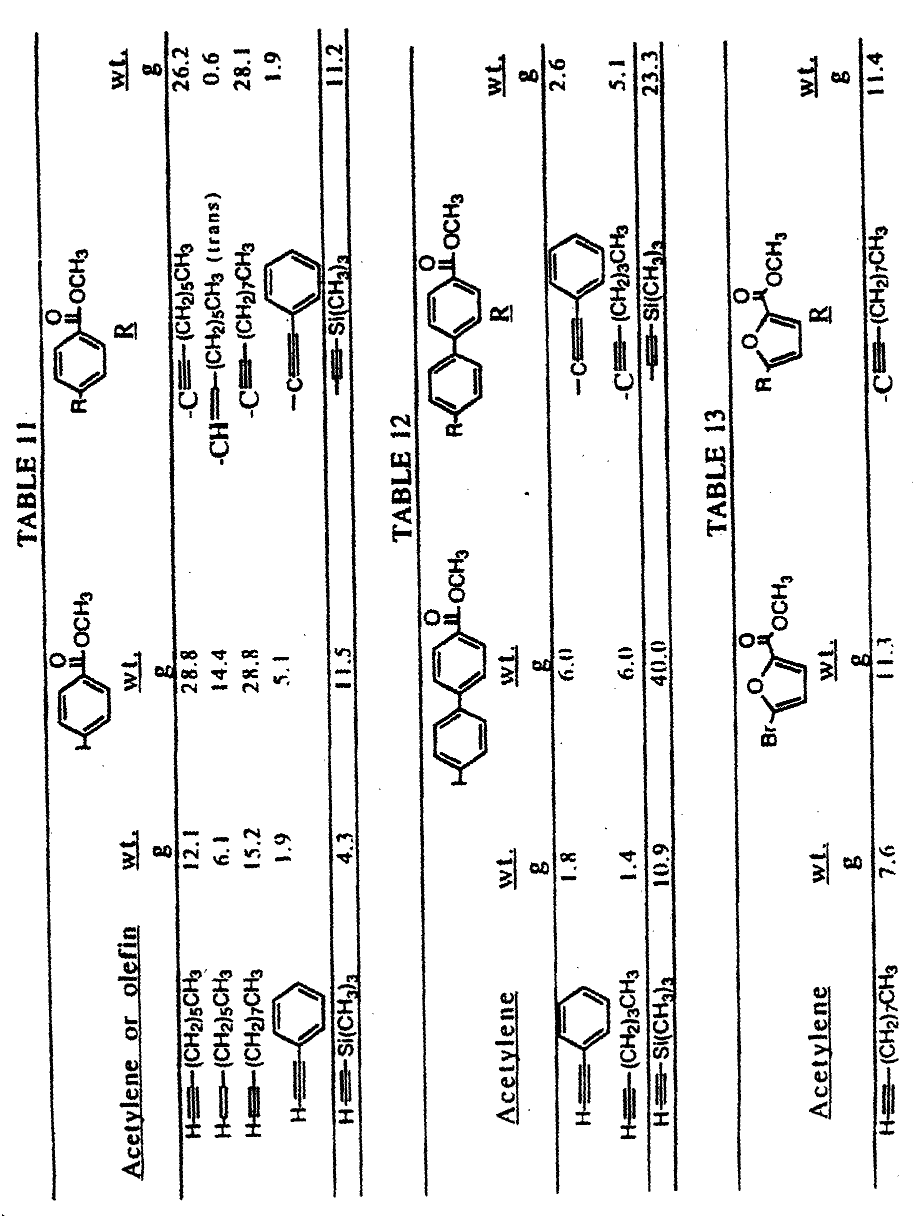

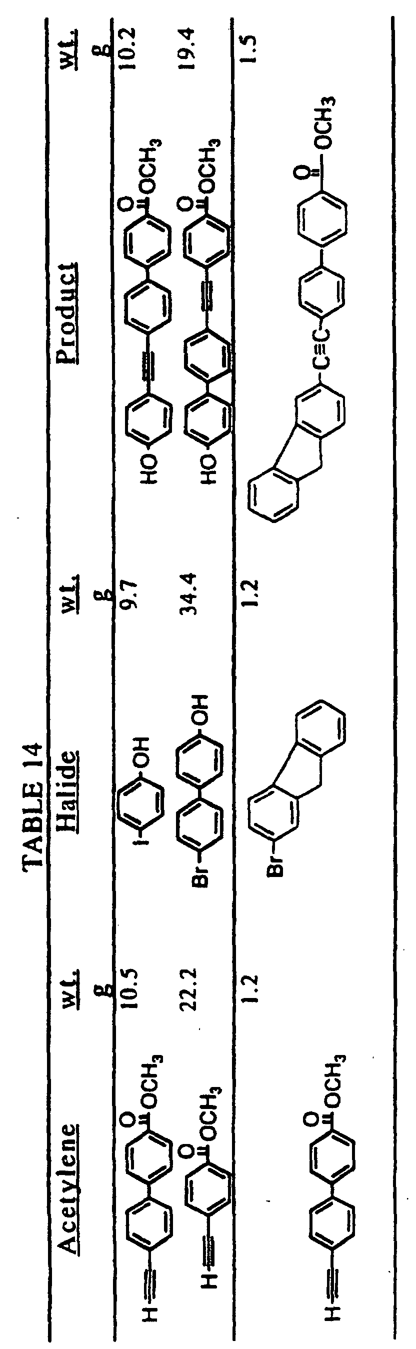

The aromatic boronic acids listed in Table 15 were prepared by the following procedure:

An aromatic halide (1 equivalent) is cooled to -78°C in tetrahydrofuran solvent. Butyl lithium (1.2 equivalents) is added. After 15 min triisopropyl borate (2 equivalents) is added and after 10 min of stirring the cooling bath is removed. When the reaction has warmed to room temperature water is added to quench the reaction followed by 1N HCl. The organic layer is removed under reduced pressure leaving a solid precipitate which is collected by filtration. This solid is washed with hexane leaving the pure boronic acid.

The terphenyl esters listed in Table 16 were made in the following manner:

An aromatic boronic acid (1 equivalent), methyl 4-iodobenzoate (1 equivalent), and potassium carbonate (1.5 equivalents) were mixed in a nitrogen-purged toluene solution. Alternatively, the trichloro phenyl ester of iodobenzoate my be used. Added tetrakis(triphenylphosphine)palladium (0.03 equivalents) and refluxed for 7 hrs. The solution was decanted to remove the potassium carbonate and reduced in vacuo. The residue was triturated with acetonitrile and the product solid was collected by filtration.

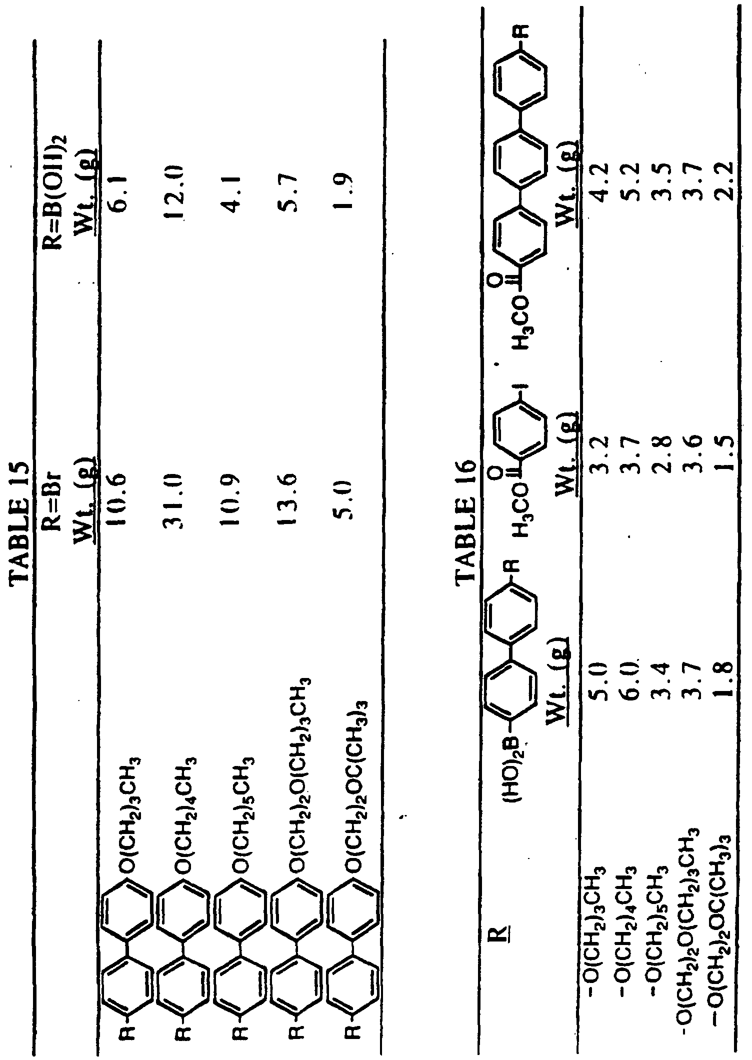

The aromatic nitriles or carboxylate esters described in Tables 9-16 can be converted to carboxylic acids by one of the two following hydrolysis procedures:
A. An aromatic nitrile is dissolved in ethanol and an excess of 50% sodium hydroxide solution and refluxed for 2 hours. Water is added until a solid precipitates. The precipitate is collected by filtration, added to dioxane and 6N hydrochloric acid solution and refluxed for 17 hours. Water is added and the carboxylic acid product crystallizes and is collected by filtration and dried under vacuum.
B. A carboxylate methyl ester is dissolved in methanol, excess 2N sodium hydroxide solution is added and the solution is refluxed for 5 hours. The solution is made acidic with excess hydrochloric acid and water is added until a precipitate forms. The carboxylic acid is collected by filtration and dried under vacuum.

The carboxylic acids are converted to 2,4,5-trichlorophenyl esters shown in Tables 18, 20 and 22-25 by the following general procedure:

The aromatic acid (1 equivalent), 2,4,5-trichlorophenol (1 equivalent), and N,N'-dicyclohexylcarbodiimide (1 equivalent) are dissolved in methylene chloride. The mixture is stirred for 17 hours after which it is filtered. The filtrate is evaporated to dryness and the residue is dissolved in ether, filtered, and pentane is added until crystallization begins. The crystalline product is collected by filtration and dried under vacuum.

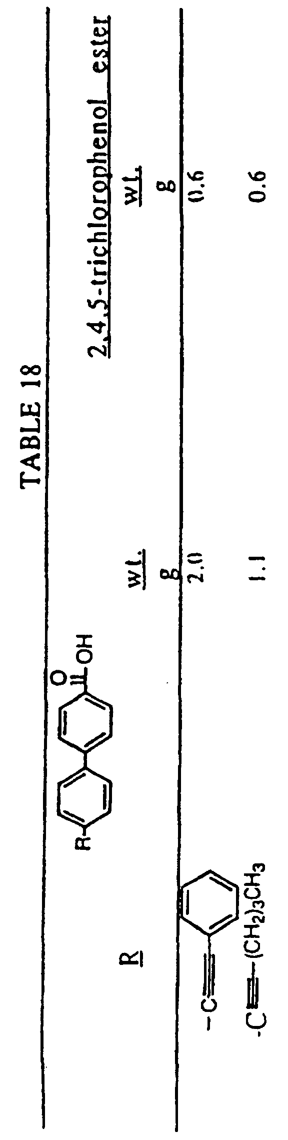

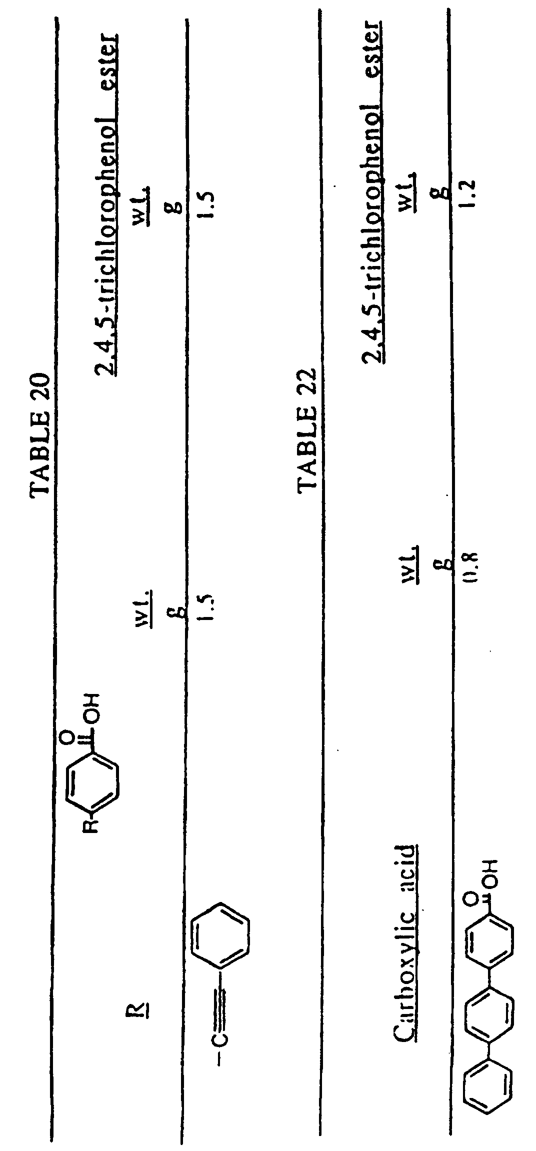

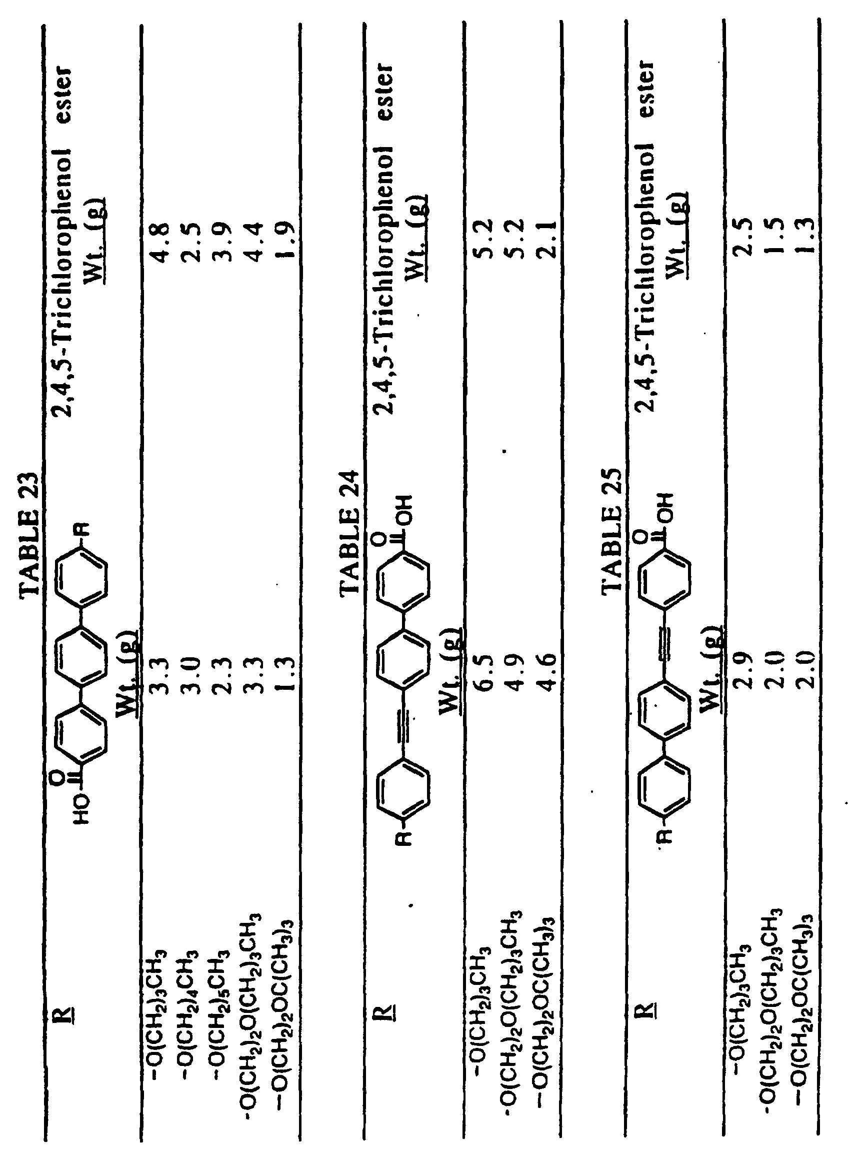

The dideoxy compounds of formula (1) are prepared by removing the benzylic and aminal hydroxy groups. The process includes subjecting a non-dideoxy compound of formula (1) (wherein R₂ may be hydrogen or acyl) to a strong acid such as trichloroacetic acid, trifluoroacetic acid or borontrifluoride etherate with trifluoroacetic acid being preferred, and a reducing agent, such as sodium cyanoborohydride or triethylsilane, with triethylsilane being preferred. The reaction takes place at temperatures of between -5 and 70°C, and in a suitable solvent such as methylene chloride, chloroform or acetic acid, with dichloromethane being preferred. The acid should be present in an amount of 2 to 60 moles per mole of substrate, and the reducing agent should be present in an amount of 2 to 60 moles per mole of substrate. This process affords selective removal of the aminal and benzylic hydroxy groups.

The compounds represented by the formula (1) have improved properties over the previously known N-acyl hexapeptide antifungals. For example, in general the compounds exhibit oral bioavailability, a property which is important for any systemic antifungal agent. Also, numerous N-acyl compounds of the formula (1) have enhanced antifungal activity and enhanced water solubility.

Among the N-acyl hexapeptides represented by the formula (1) certain are preferred embodiments of the invention. The compounds wherein R₂ is a diphenyl acyl group 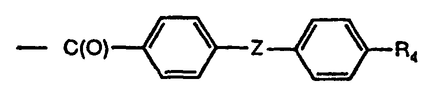 wherein Z is a carbon to carbon bond and R₄ is -Y-R₆ and R₆ is C₁-C₁₂ alkyl phenyl or substituted phenyl and Y is an acetylenic bond.

Examples of preferred compounds of the above mentioned group include compounds wherein R₄ is 4-[4-(phenylethynyl)-phenyl]benzoyl and 4-[4-(n-butylethynyl)phenyl]benzoyl.

Preferred cyclohexylpeptide compounds are represented by the formula 1 wherein R'=R''= methyl, R₁ is hydrogen and R₂ is a preferred acyl group as defined hereinabove.

Table 26 is a list of the most preferred R₂ substituents, wherein R=R₇=R^{y}=OH; R'=R''= R'''=CH₃; and R₁=H.

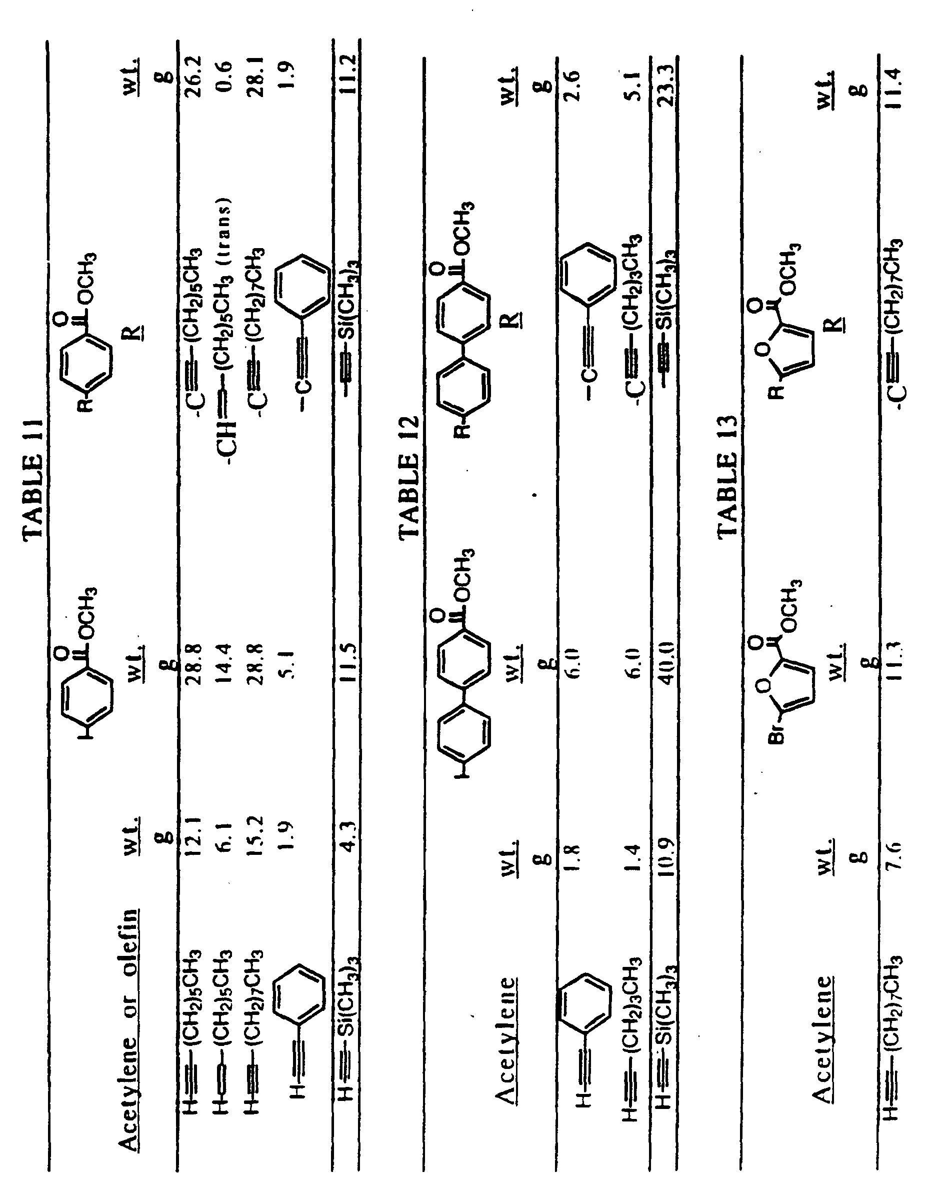

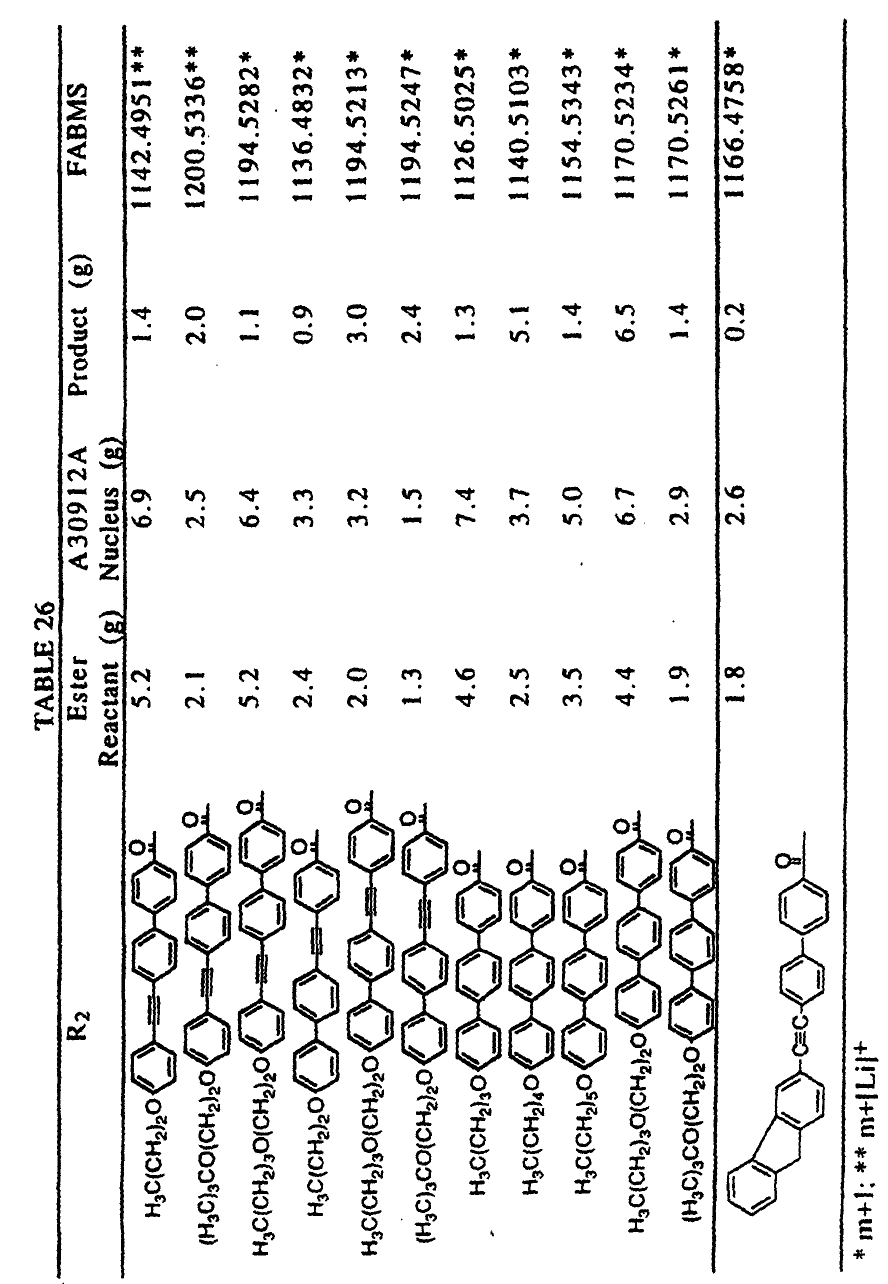

The N-acylhexapeptides provided by this invention are useful in the treatment of fungal infections both systemic infections and skin infections. Accordingly this invention also provides a method for treating fungal infections in man and animals which comprises administering to said host an antifungally effective non-toxic amount of an N-acyl-cyclohexapeptide represented by the formula 1. A preferred antifungal method comprises administering an N-acylhexapeptide compound where, in formula 1, R'=R''= methyl, R₁ is hydrogen and R₂ is a preferred acyl group as defined hereinabove.

The antifungal compound can be administered parenterally, e.g. i.m., i.p. or s.c., nasally, orally or can be applied topically for skin infections. The dose administered of course will vary depending on such factors as the nature and severity of the infection, the age and general health of the host and the tolerance of a particular host to the particular antifungal agent. The particular dose regimen likewise may vary according to such factors and may be given in a single daily dose or in multiple doses during the day. The regimen may last from about 2-3 days up to about 2-3 weeks or longer.

This invention also provides pharmaceutical formulations useful for administering the antifungal compounds of the invention. These formulations comprise an N-acylhexapeptide represented by the formula 1 or a pharmaceutically acceptable, non-toxic salt thereof and a pharmaceutically acceptable carrier.

For parenteral administration the formulation comprises a compound of the formula 1 and a physiologically acceptable diluent such as deionized water, physiological saline, 5% dextrose and other commonly used diluents. The formulation may contain a solubilizing agent such as a polyethylene glycol or polypropylene glycol or other known solubilizing agent. Such formulations may be made up in sterile vials containing the antifungal and excipient in a dry powder or lyophilized powder form. Prior to use, the physiologically acceptable diluent is added and the solution withdrawn via syringe for administration to the patient. For oral administration, the antifungal compound is filled into gelatin capsules or formed into tablets. Such tablets also contain a binding agent, a dispersant or other suitable excipients suitable for preparing a proper size tablet for the dosage and particular antifungal compound of the formula 1. For pediatric or geriatric use the antifungal compound may be formulated into a flavored liquid suspension, solution or emulsion. A preferred oral carrier system is lineolic acid, cremophor RH-60 and water and preferably in the amount (by volume) of 8% lineolic acid, 5% cremophor RH-60, and 87% sterile water. The compound is added to the system in an amount of 2.5 to 40 mg/ml.

For topical use the antifungal compound can be formulated with a dry powder for application to the skin surface or it may be formulated in a liquid formulation comprising a solubilizing aqueous liquid or non-aqueous liquid, e.g., an alcohol or glycol. Such formulations are useful forms for use in the antifungal method provided herein.

The N-acylcyclohexapeptides provided herein may be formulated as described above in unit dosage formulations comprising for injection between about 50 mg and about 500 mg per vial. For oral use gelatin capsules or tablets comprising between about 100 mg and about 500 mg per capsule or tablet can be provided.

Preferred formulations of the invention comprises the active ingredient presented by the formula 1 wherein R'=R''= methyl, R₁ is hydrogen and R₂ is 4-[4-(phenylethynyl)-phenyl]benzoyl in gelatin capsules or as active ingredient the antifungal represented by the formula 1 wherein R'=R''= methyl, R₁ is hydrogen and R₂ is 4-[4-[2-(4-cyclohexyl-piperidino)ethoxy]phenyl]benzoyl or the hydrochloride salt form thereof in tablet or gelatin capsules. Further preferred formulations are those in which a preferred compound, as described above, is employed.

In yet a further aspect of the present invention there is provided a method for treating patients suffering from Pneumocystis pneumonia. The method can be used prophylactically to prevent the onset of the infection which is caused by the organism Pneumocystis carinii. The N-acylcyclicpeptide can be administered parenterally, e.g. via intramuscular (i.m), intravenous (iv.) or intraperitoneal (i.p.) injection, or orally or by inhalation directly into the airways of the lungs. Preferably the cyclic peptide is administered via inhalation of an aerosol spray formulation of the compound.

An effective amount of a cyclic peptide will be between about 3 mg/kg of patient body weight to about 100 mg/kg. The amount administered may be in a single daily dose or multiple doses e.g. two, three or four times daily throughout the treatment regimen. The amount of the individual doses, the route of delivery, the frequency of dosing and the term of therapy will vary according to such factors as the intensity and extent of infection, the age and general health of the patient, the response of the patient to therapy and how well the patient tolerates the drug. It is known that PCP infections in AIDS patients are highly refractory owing to the nature of the infection. For example, in severe, advanced infections the lumenal surface of the air passages becomes clogged with infectious matter and extensive parasite development occurs in lung tissue. A patient with an advanced infection will accordingly require higher doses for longer periods of time. In contrast, immune deficient patients who are not severely infected and who are susceptible to PCP can be treated with lower and less frequent prophylactic doses.

The activity of the cyclicpeptide represented by the formula 1 is demonstrated in immunosuppressed rats. The tests were carried out in general as follows. One week after initiation of immunosuppression rats were inoculated intratracheally with parasites and maintained on immunosuppression for the remainder of the study. Prophylactic treatments began one day after parasite inoculation and therapeutic treatments began 3 or 4 weeks later after moderate PCP developed. Eight or ten animals were assigned to the following groups: those receiving test compound; non-treated Pneumocystis infected control animals; animals treated with trimethoprim-sulfamethoxazole (TMP-SMX); or non-treated, non-infected control animals. The efficacy of different treatments was evaluated by monitoring animal weights and survival during the studies and by determining the severity of PCP at necropsy. Stained impression smears of the lungs and stained lung homogenates were evaluated to determine the intensity of P. carinii infection.

The immune deficient rats employed in the tests were prepared as follows. Female Lewis rats weighing from 120-140 g each were immune suppressed with methyl prednisolone acetate at a dose of 4 mg/100 g for the first week, 3 mg/100 g for the second week and continuing weekly thereafter at 2 mg/100 g. All rats, except for the non-infected control rats, were inoculated intratracheally with 0.1 ml to 0.2 ml of Dulbecco's Modified Eagle Media containing between >10⁵ and 10⁶ P. carinii (trophozoites, precysts and cysts) harvested from the lungs of heavily infected donor animals (infection scores of 6) and maintained as cryopreserved (liquid nitrogen) inocula. Rats were maintained on immune suppression and PCP was allowed to develop for 3 or 4 weeks before initiation of therapy with test compounds. Body weights were recorded weekly and rats were allocated into treatment groups such that each group had a similar distribution of percent weight loss among animals. Rats were treated with test compounds for 2 or 3 weeks and then were necropsied. For prophylaxis studies, administration of test compound was initiated one day after intratracheal inoculation of parasites and was continued until the rats were necropsied.

Following the evaluation period for test compounds, the rats were necropsied and test results evaluated by Giemsa-stained, silver-methenamine stained impression smears and/or by silver-methenamine stained lung homogenate (see below). Necropsy was carried out as follows. The test rats were anesthetized with a mixture of ketamine hydrochloride and xylazine and then exsanguinated via the right atrium. Internal organs in the abdominal and thoracic cavities were examined for gross lesions.

A small portion of lung tissue from the left lobe of each rat was used to make the impression smears described below. Giemsa-stained impression smears were evaluated to determine the total number of parasites (trophozoites, precysts, and cysts). Impression smears from rats in groups whose treatments exhibited some anti-Pneumocystis activity (as judged by infection scores from Giemsa-stained slides) and from rats in the control groups were also stained with methamine silver, a stain specific for the cyst wall of the organism. Impression smears were randomized, numbered, and then evaluated. The infection scores used were as follows:

| Score | Basis |
|---|---|
| O | No parasites found |
| 1 | 1 to 5 parasites/10 oil fields |
| 2 | ca 1 parasite/field |
| 3 | 2-10 parasites/field |
| 4 | >10 but <100 parasites/field |
| 5 | >100 but <1,000 parasites/field |

A score of 6 was reserved for those infections with impression smears containing >1,000 organisms/field (too numerous to count). Giemsa-stained slides were examined microscopically using a final magnification of 1008X. Methenamine silver-stained slides were examined with a final magnification of 400X.

Cysts in rat lung tissue were quantified as follows. A small portion of lung tissue from the left lobe of each rat was used to make impression smears as described above. The remainder of each lung was weighed, placed in a tube containing Hanks balanced salt solution (HBSS) (40X the lung weight) and homogenized using a Brinkman model tissue homogenizer. Two µl samples of the homogenized lung samples (1:4 dilution in HBSS) were placed in wells of teflon-coated, 12-well slides, stained with methenamine silver, and the number of cysts were scored as described above for the impression smears.

The activity and efficacy of a preferred N-acylcyclohexapeptide in the test animals is presented below. The compound of the formula 1 wherein R'=R''= methyl, R₁ is hydrogen and R₂ is 4[(4-phenylethynyl)phenyl]benzoyl when administered as an aerosol solution at a concentration of 5 mg/ml for one hour, twice weekly for 5 weeks resulted in 90% reduction in P. carinii cysts in the lungs. When given orally at 10 mg/kg, bid for 3 weeks, the number of cysts in the lungs was reduced by >99% when compared with infected vehicle controls.

When the preferred N-acylcyclicpeptides were administered orally and by intraperitoneal injection the compound was effective in clearing P. carinii cysts from the lungs of heavily infected rats. For example, when the compound was administered at 10 or 40 mg/kg, bid for 4, 8 or 12 days, the number of identifiable cysts in the lungs of heavily infected rats was reduced by >99%. Similar efficacy was observed when the compound was administered i.p. at 1 mg/kg.

When tested orally for prophylactic activity, the preferred compound exhibited >99% cyst reduction in one of two studies when infected animals were dosed at 1 mg/kg and when given higher doses of 5 or 4 mg/kg.

The following examples of compounds of the invention and the manner of their preparation further describe the present invention.

### N-Acylation of Cyclohexpeptide Nuclei

The preparation of the derivatives of the A30912A nucleus was accomplished by the following general procedure, with Table 27 listing these derivatives.

The A30912A nucleus and the 2,4,5-trichlorophenol ester are dissolved in dimethylformamide (25-50 ml) and stirred for 17-65 hours at room temperature. The solvent is removed *in vacuo* and the residue is slurried in ether and collected by filtration. The solid product is washed with methylene chloride and then dissolved in either methanol or acetonitrile/water (1:1 v/v). This solution is injected on a waters 600E semi-preparative chromatography system using a Rainin Dynamax-60A C₁₈ reverse-phase column. The column is eluted beginning with 20-40% aqueous acetonitrile and 0.5% monobasic ammonium phosphate (w/v) (monitored by UV at 230 nm and at a flow rate of 20 ml/min) until the unreacted A30912A nucleus is eluted and then deleting the buffer and eluting the product peak in aqueous acetonitrile. The fraction containing the product is evaporated in vacuo or lyophilized to provide the pure compound. The product may be analyzed by the same HPLC instrument using a Waters C₁₈ Micro Bondapak column and eluting with 40% aqueous acetonitrile containing 0.5% monobasic ammonium phosphate (w/v) at a 2 ml/min flow rate and monitoring the UV at 230 nm. The products may also be analyzed by fast atom bombardment mass spectrometry (FABMS). (In the compounds used, R'=R''=R'''=CH₃, R=OH, R^{y}=OH, R₁=H, R₇=OH, and R₂ is as defined).

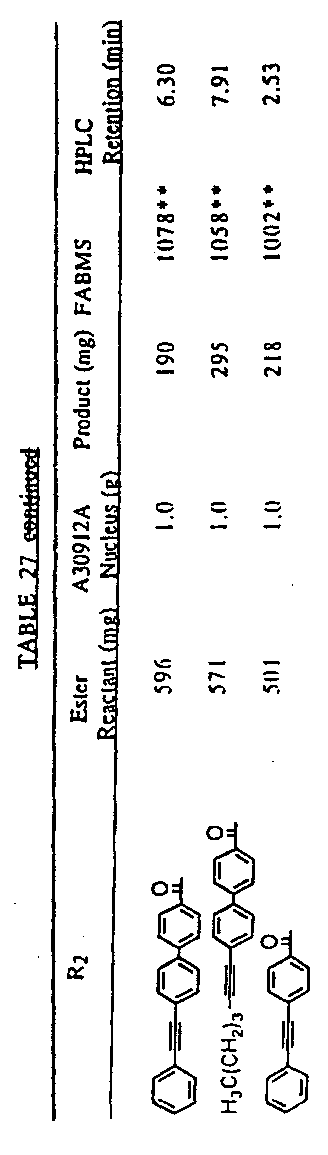

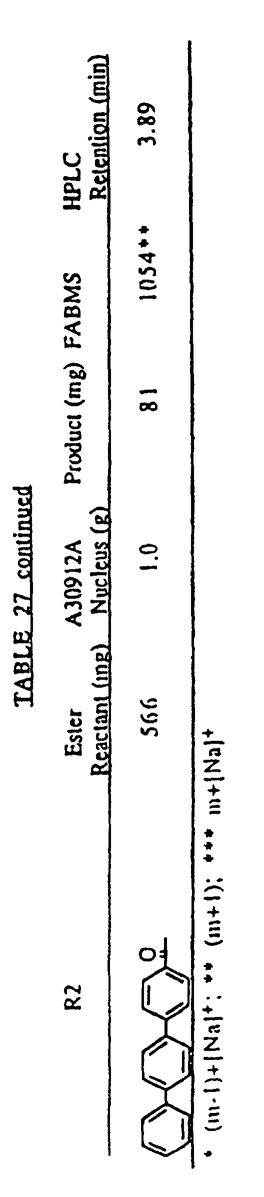

Compounds such as those listed in Table 27 could be further modified at the phenolic hydroxy to provide R₇ = -OPO₃HNa as shown in Table 28. The procedure is as follows:

The lipopeptide (1 equivalent) and tetrabenzylpyrophosphate (2 equivalents) were dissolved in dimethylformamide which had been dried over 13X molecular sieves. Lithium hydroxide monohydrate (5 equivalents) was added and the stirred solution was monitored by HPLC. After 0.5 hr and 1 hr more lithium hydroxide (5 equivalents) was added. Between 1 and 2 hrs. the reaction was quenched with glacial acetic acid, the solvent removed under vacuum, and the residue purified over a semi-preparative C18 reverse-phase column using an aqueous acetonitrile eluent. The purified product was dissolved in (1/1) acetic acid/water with sodium acetate (1 equivalent) and 10% Pd/C catalyst. The solution was placed under an atmosphere of hydrogen gas and stirred for 1 hr. After filtering to remove the catalyst, the solution was lyophilized to provide the pure final product. The purity was assessed by analytical HPLC and the product was analyzed by fast atom bombardment mass spectrometry (FABMS).

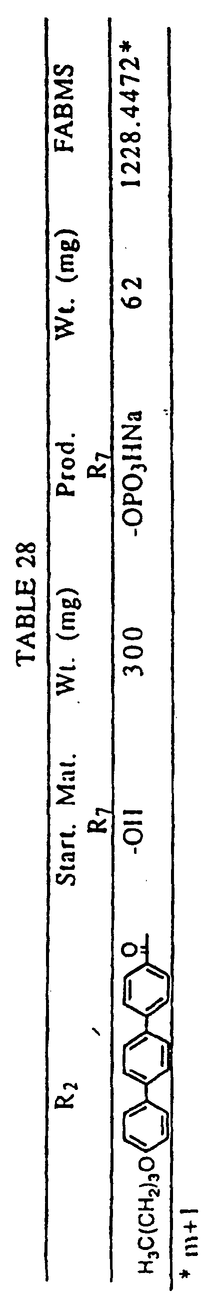

### Preparation of dideoxy cyclohexapeptide

The preparation of the dideoxy compounds may be accomplished by the following procedure.

To a suspension of a non-dideoxy cyclohexapeptide (formula (I) where R=OH and R₂ is hydrogen or acyl), in dichloromethane is added the reducing agent triethylsilane in dichloromethane. The solution is stirred and the volatile components are removed under reduced pressure and the residue triturated with diethyl ether. The compound is purified using HPLC, and the product lyophilized.

### Example

### Dideoxycilofungin

To a suspension of cilofungin (10.00 g, 9.71 mmol) in dichloromethane (100 ml) was added a solution of triethylsilane (96 ml, 602 mmol) in dichloromethane (50 ml). Trifluoroacetic acid (46.4 ml, 602 mmol) was added as a solution in dichloromethane (50 ml) over 15 minutes. The solution was stirred at room temperature for two hours. The volatile reaction components were removed under reduced pressure and the residue triturated with diethyl ether. The compound was purified by reversed phase HPLC by means of a "Prep LC/System 500" unit (Waters Associates, Inc., Milford, Mass.) using a Prep Pak 500/C₁₈ Column (Waters Associates, Inc.) as the stationary phase. The column eluted with a gradient mobile phase using CH₃CN/H₂O (10:90 to 20:80 v/v) at 500 psi. The product containing fractions were pooled, evaporated under reduced pressure, and lyophilized from p-dioxane to yield dideoxycilofungin (6.66 g, 68.7%). FAB-MS: m/z calc. for C₄₉H₇₂N₇O₁₅, 998.5086; found, 998.512; UVλ(EtOH)nm(ε) 202.60(61012), 256.20(18569).

A compound of the formula 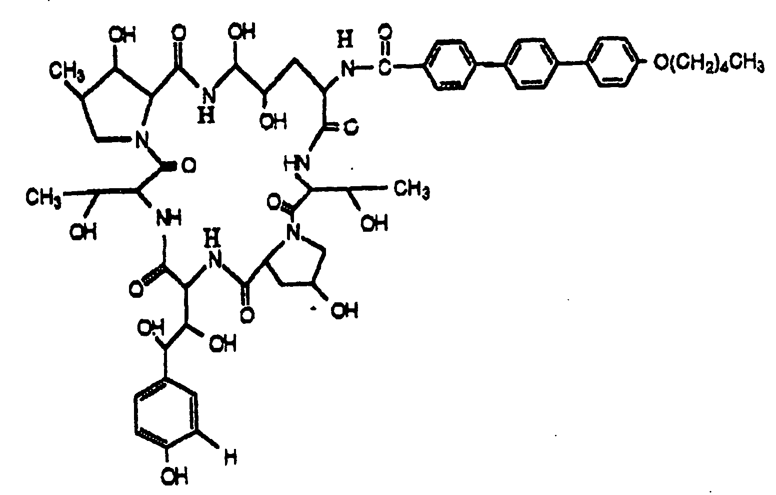 the preparation of which is discussed just prior to Table 27, can also be further modified at the phenolic hydroxy to provide R₇=-OPO₃HNa, as indicated in the two paragraphs prior to Table 28. The compound produced is as follows: 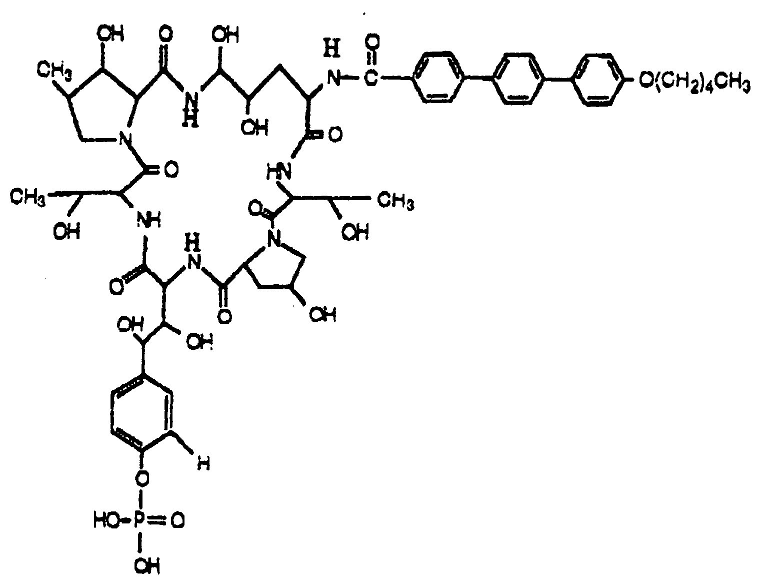 The product was analyzed by FABMS (using Lit) to give a peak at 1226.4853 (calculated for C₅₈H₇₄N₇O₂₀PLi=1226.4886). Also, when analyzed by HPLC using a C18 reverse-phase column and eluting with 55% aqueous acetonitrile with 0.5% acetic acid at 2 ml/min and monitoring by UV at 280 nm, the compound had a retention time of 1.72 min.

## Claims

1. A compound of the formula (1): 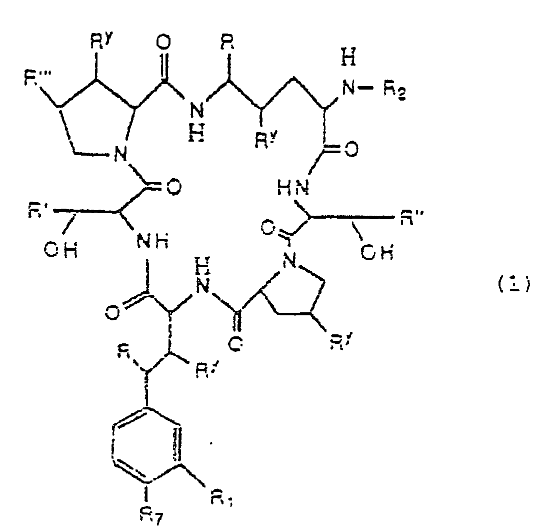 wherein
R' is hydrogen, methyl or NH₂C(O)CH₂-;
R" and R"' are independently methyl or hydrogen;
R and R^{y} are independently hydroxy or hydrogen;
R₁ is hydroxy, hydrogen or hydroxysulfonyloxy;
R₇ is hydroxy, hydrogen, hydroxysulfonyloxy or phosphonooxy; and
I) R₂ is a substituted benzoyl group represented by the formula 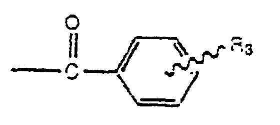 wherein R₃ is quinolyl; or
(II) R₂ is an acyl group represented by the formula 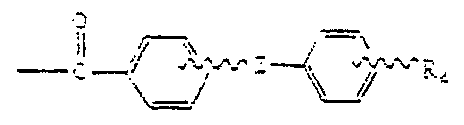 wherein
Z is -C≡C-, -CH=CH-, or a carbon to carbon bond;
(A) R₄ is C₃-C₁₂ cycloalkyl, C₇-C₁₀ bicycloalkyl, C₇-C₁₄ tricycloalkyl, C₃-C₁₂ cycloalkoxy, naphthyl, pyridyl, thienyl, benzothienyl, quinolyl or phenyl; or
(B) R₄ is phenyl substituted by amino, C ₁-C₁₂ alkylthio, halogen, C₁-C₁₂ alkyl, C₂-C₁₂ alkenyl, C₂-C₁₂ alkynyl, C₁-C₁₂ substituted alkyl, C₂-C₁₂ substituted alkenyl, C₂-C₁₂ substituted alkynyl, C₁-C₁₂ alkoxy, trifluoromethyl, phenyl, substituted phenyl, phenyl substituted with a polyoxa-alkyl group represented by the formula
-O-(CH₂)ₘ-[O-(CH₂)ₙ]ₚ-O-(C₁-C₁₂ alkyl)
wherein m and n are integers of from 2 to 4, and p is 0 or 1; or
C) R₄ is phenyl substituted with C₁-C₆ alkoxy substituted by fluoro, bromo, chloro or iodo; or
D) R₄ is a group represented by the formula
-Y-R₆
wherein
Y is -C≡C- or -C=C-; and
R₆ is C₁-C₁₂ alkyl, C₁-C₁₂ substituted alkyl; C₃-C₁₂ cycloalkyl, C₇-C₁₀ bicycloalkyl, C₇-C₁₄ tricycloalkyl, phenyl, C₃-C₁₂ cycloalkenyl, naphthyl, benzothiazolyl, thienyl, phenyl substituted by amino, C₁-C₁₂ alkylthio, halogen, C₁-C₁₂ alkyl, C₂-C₁₂ alkenyl, C₂-C₁₂ alkynyl, C₁-C₁₂ alkoxy, trifluoromethyl, -O-(CH₂)_{p'}-W-R₅ wherein p' is an integer of from 2 to 4; W is pyrrolidino, piperidino or piperazino, and R₅ is hydrogen, C₁-C₁₂ alkyl, C₃-C₁₂ cycloalkyl, benzyl or C₃-C₁₂ cycloalkylmethyl; or C₁-C₆ alkoxy substituted by fluoro, bromo, iodo or chloro; or
R₆ is a phenyl substituted by a polyoxa-alkyl group represented by the formula
-O-(CH₂)ₘ-[O-(CH₂)ₙ]ₚ-O-(C₁-C₁₂ alkyl)
wherein m, n and p are as defined above; or
R₂ is an acyl group represented by the formula 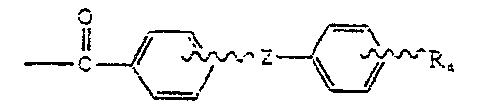
wherein
Z is -C≡C- or -CH=CH-;
A) R₄ is hydrogen, C₂-C₁₂ alkynyl, C₂-C₁₂ substituted alkynyl, C₁-C₁₂ alkoxy; or
B) R₄ is C₁-C₁₂ alkoxy substituted with C₃-C₁₂ cycloalkyl, C₇-C₁₀ bicycloalkyl, C₇-C₁₄ tricycloalkyl, C₂-C₁₂ alkynyl, amino, C₁-C₄ alkylamino, di-(C₁-C₄ alkyl)amino, C₁-C₁₂ alkanoylamino, phenyl substituted with a polyoxa-alkyl group represented by the formula
-O-(CH₂)ₘ-[O-(CH₂)ₙ]ₚ-O-(C₁-C₁₂ alkyl)
wherein m,n and p are as defined; or
C) R₄ is C₁-C₁₂ alkoxy substituted with a group of the formula 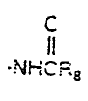 wherein R₃ is C₁-C₆ alkoxy optionally substituted with phenyl; or
D) R₄ is a group represented by the formula
-O-(CH₂)_{p'}-W-R₅
wherein p', W and R₅ are as defmed; or
IV) R₂ is a group having the formula 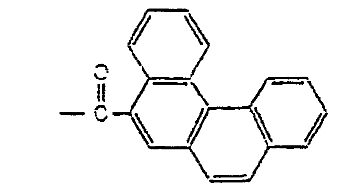 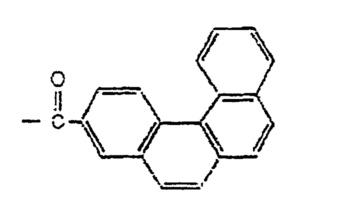 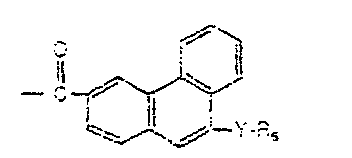 wherein Y and R₆ are as defined above; or
V) R₂ is naphthoyl substituted with R₄
wherein
A) R₄ is C₃-C₁₂ cycloalkyl, C₇-C₁₀ bicycloalkyl, C₇-C₁₄ tricycloalkyl, C₃-C₁₂ cycloalkoxy, naphthyl, pyridyl, thienyl, benzothienyl, quinolyl or phenyl; or
B) R₄ is phenyl substituted by amino, C₁-C₁₂ alkylthio, halogen, C₁-C₁₂ alkyl, C₂-C₁₂ alkenyl, C₂-C₁₂ alkynyl, C₁-C₁₂ substituted alkyl, C₂-C₁₂ substituted alkenyl, C₂-C₁₂ substituted alkynyl, C₁-C₁₂ alkoxy, trifluoromethyl, phenyl, substituted phenyl, phenyl substituted with a polyoxa-alkyl group represented by the formula
-O-(CH₂)ₘ-[O-(CH₂)ₙ]ₚ-O-(C₁-C₁₂ alkyl)
wherein m, n and p are as defined; or
C) R₄ is phenyl substituted with C₁-C₆ alkoxy substituted by fluoro, bromo, chloro or iodo; or
D) R₄ is a group represented by the formula
-Y-R₆
wherein Y has the same meanings as defined above; and
R₆ is C₁-C₁₂ alkyl, C₁-C₁₂ substituted alkyl; C₃-C₁₂ cycloalkyl, C₇-C₁₀ bicycloalkyl, C₇-C₁₄ tricycloalkyl, phenyl, C₃-C₁₂ cycloalkenyl, naphthyl, benzothiazolyl, thienyl, phenyl substituted by amino, C₁-C₁₂ alkythio, halogen, C₁-C₁₂ alkyl, C₂-C₁₂ alkenyl, C₂-C₁₂ alkynyl, C₁-C₁₂ alkoxy, trifluoromethyl, -O-(CH₂)_{p'}-W-R₅, or C₁-C₆ alkoxy substituted by fluoro, bromo, iodo or chloro; or
R₆ is a phenyl substituted by a polyoxa-alkyl group represented by the formula
-O-(CH₂)ₘ-[O-(CH₂)ₙ]ₚ-O-(C₁-C₁₂ alkyl)
wherein m, n and p are as defined above; and the pharmaceutically acceptable non-toxic salts thereof.

2. A compound according to claim 1 wherein R₁ is hydroxy or hydrogen
and R₇ is hydroxy or hydrogen.

3. A compound according to claim 1 or claim 2 wherein R', R" and R'" are methyl, R₁ is hydrogen, and R₇ and R^{y} are OH.

4. A compound according to any one of claims 1 to 3 wherein R₂ is of the formula 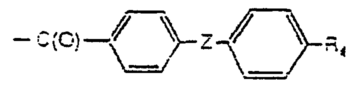 wherein
Z is a carbon to carbon bond; and
R₄ is C₃-C₇ cycloalkoxy; or
R₄ is phenyl substituted by C₁-C₁₂ alkoxy or phenyl substituted with a polyoxa-alkyl group of the formula
-O-(CH₂)ₘ-[O-(CH₂)ₙ]ₚ-O-(C₁-C₁₂ alkyl);
or
R₄ is a group of the formula -Y-R₆, wherein Y is -C≡C- or -C=C- and R₆ is C₁-C₆ alkyl, phenyl, or phenyl substituted with a polyoxa-alkyl group of the formula
-O-(CH₂)ₘ-[O-(CH₂)ₙ]ₚ-O-(C₁-C₁₂ alkyl).

5. A compound according to any one of claims 1 to 3 wherein R₂ is of the formula 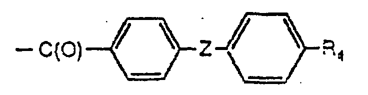 wherein
Z is -C≡C-; and
R₄ is phenyl substituted by C₁-C₁₂ alkoxy or phenyl substituted with a polyoxa-alkyl group of the formula
-O-(CH₂)ₘ-[O-(CH₂)ₙ]ₚ-O-(C₁-C₁₂ alkyl)
or
R₄ is a group of the formula
-O-(CH₂)_{p'}-W-R₅
wherein W is a piperidine group.

6. A compound according to any one of claims 1 to 3 wherein R is hydrogen.

7. A compound according to any one of claims 1 to 3 wherein R₂ is 4-[4-(phenylethynyl)phenyl]benzoyl or 4-[4-(n-butylethynyl)phenyl]beraoyl.

8. A compound of the formula (1): 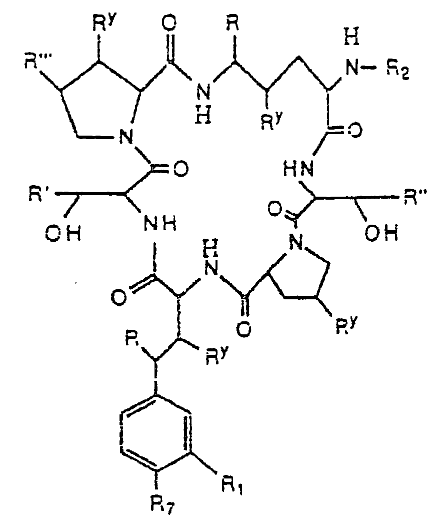 wherein
R' is hydrogen, methyl or NH₂C(O)CH₂-;
R" and R"' are independently methyl or hydrogen;
R and R^{y} are independently hydroxy or hydrogen;
R₁ is hydroxy, hydrogen, or hydroxysulfonyloxy;
R₇ is hydroxy, hydrogen, hydroxysulfonyloxy or phosphonooxy; and
I) R₂ is a group of the formula 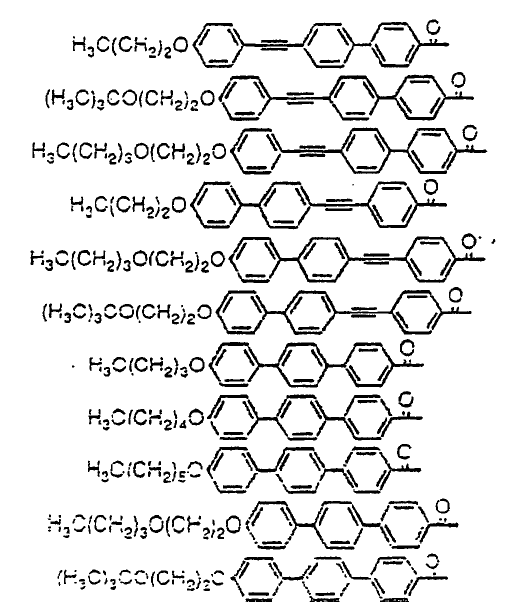 and pharmaceutically acceptable salts thereof.

9. A compound according to claim 8 wherein R', R" and R"' are methyl, R₁ is hydrogen and R₇ and R^{y} are hydroxy.

10. A compound of the formula (1): 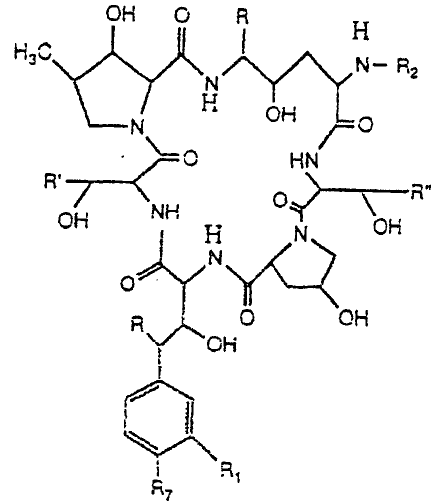 wherein
R' is hydrogen, methyl or NH₂C(O)CH₂-;
R" is methyl or hydrogen;
R is hydroxy or hydrogen;
R₁ is hydroxy, hydrogen, or hydroxysulfonyloxy;
R₇ is hydroxy, hydrogen, hydroxysulfonyloxy or phosphonooxy;
R₂ is an acyl group represented by the formula 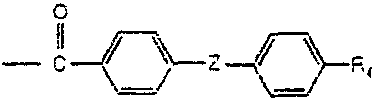 wherein Z is -C≡C-, -CH=CH-, or a carbon to carbon bond;
R₄ is C₃-C₁₂ cycloalkyl, C₇-C₁₀ bicycloalkyl, C₇-C₁₄ tricycloalkyl, phenyl, phenyl substituted by amino, C₁-C₁₂ alkythio, halogen, C₁-C₁₂ alkyl, C₁-C₁₂ alkoxy, trifluoromethyl, phenyl, or C₁-C₆ alkoxy substituted by fluoro, bromo, chloro or iodo;
or R₄ is C₃-C₁₂ cycloalkoxy;
or R₄ is a group represented by the formula -Y-R₆ wherein Y is -C≡C- or -CH=CH- and R₆ is C₁-C₁₂ alkyl, C₁-C₁₂ alkyl substituted by phenyl; C₃₋C₁₂ cycloalkyl, phenyl, C₃₋C₁₂ cycloalkenyl, naphthyl, benzthiazol-2-yl, or phenyl substituted by amino, C₁-C₁₂ alkythio, halogen, C₁-C₁₂ alkyl, C₁-C₁₂ alkenyl. C₁-C₁₂ alkoxy, trifluoromethyl, -O-(CH₂)_{p'}-W-R₅ wherein p' is an integer of from 2 to 4; W is pyrrolidino, piperidino or piperazino, and R₅ is hydrogen, C₁-C₁₂ alkyl, C₃-C₁₂ cycloalkyl, benzyl or C₃-C₁₂ cycloalkylmethyl; or C₁-C₆ alkoxy substituted by fluoro, bromo, iodo or chloro;
or R₂ is an acyl group represented by the formula 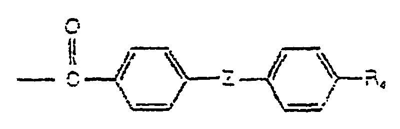 wherein
Z is -C≡C- or -CH=CH-;
R₄ is hydrogen:
R₄ is C₁-C₁₂ alkoxy, C₁-C₁₂ alkoxy substituted by C₃-C₁₂ cycloalkyl, C₇-C₁₀ bicycloalkyl, C₇-C₁₄ tricycloalkyl, amino, C₁-C₄ alkylamino, di-(C₁-C₄ alkyl)amino, C₁-C₁₂ alkanoylamino or a group of the formula 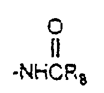
wherein R₈ is C₁-C₆ alkoxy optionally substituted with phenyl; or R₄ is a group represented by the formula
-O-(CH₂)_{p'}-W-R₅
or
R₂ is a group selected from 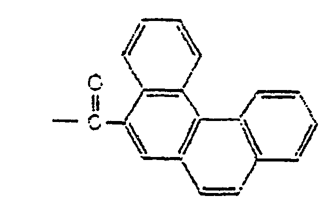 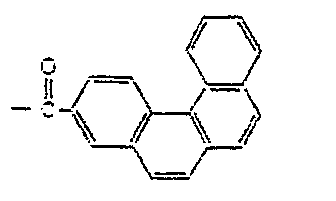 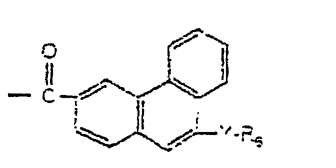 wherein
Y and R₆ are as defined above; or
R₂ is naphthoyl substituted with R₄ wherein
R₄ is C₃-C₁₂ cycloalkyl, C₇-C₁₀ bicycloalkyl, C₇-C₁₄ tricycloalkyl, phenyl, phenyl substituted by amino, C₁-C₁₂ alkythio, halogen, C₁-C₁₂ alkyl, C₁-C₁₂ alkoxy, trifluoromethyl, phenyl, or C₁-C₆ alkoxy substituted by fluoro, bromo, chloro or iodo;
or R₄ is C₃-C₁₂ cycloalkoxy;
or R₄ is a group represented by the formula -Y-R₆ wherein Y has the same meanings as defined above and R₆ is C₁-C₁₂ alkyl, C₁-C₁₂ alkyl substituted by phenyl; C₃-C₁₂ cycloalkyl, phenyl, C₃-C₁₂ cycloalkenyl, naphthyl, benzthiazol-2-yl, or phenyl substituted by amino, C₁-C₁₂ alkylthio, halogen, C₁-C₁₂ alkyl, C₁-C₁₂ alkenyl, C₁-C₁₂ alkoxy, trifluoromethyl, -O-(CH₂)_{p'}-W-R₅, or C₁-C₆ alkoxy substituted by fluoro, bromo, iodo or chloro; and the pharmaceutically acceptable non-toxic salts thereof.

11. A compound according to claim 10 wherein R₁ is not hydroxysulfonyloxy and R₇ is not hydroxysulfonyloxy or phosphonooxy.

12. A compound of the formula

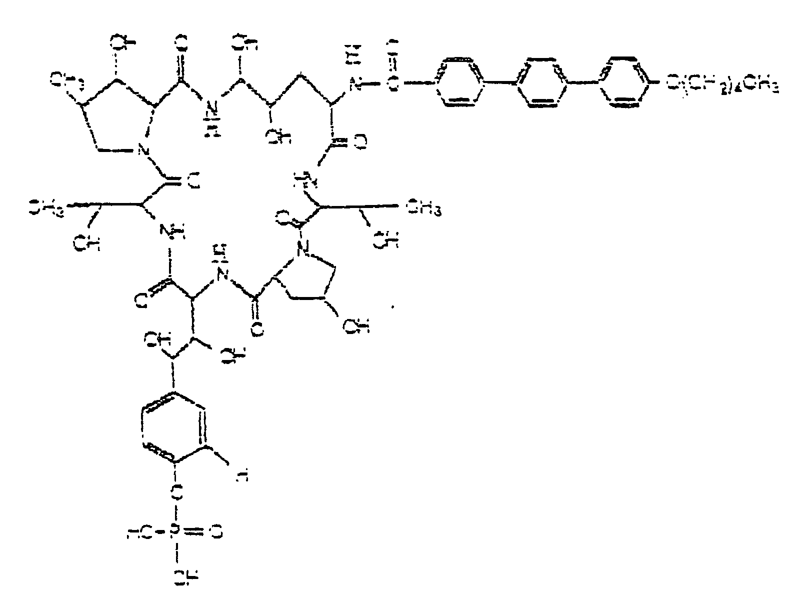

13. A compound of the formula

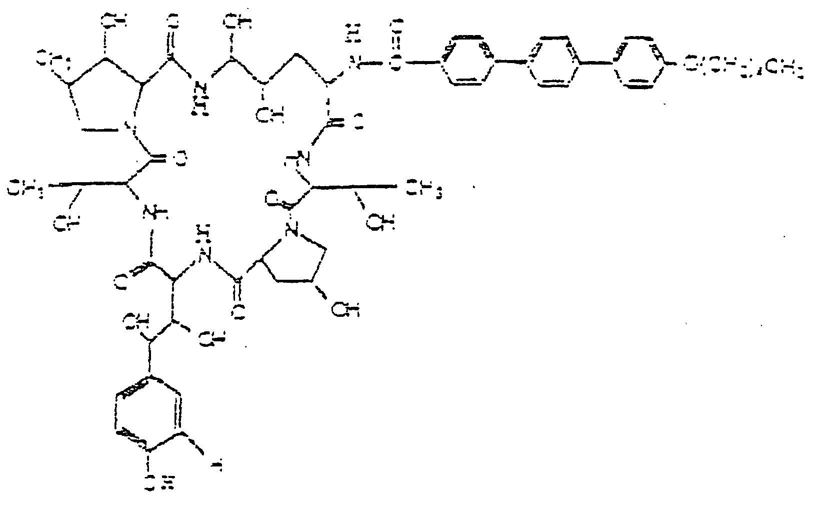

14. A compound of the formula 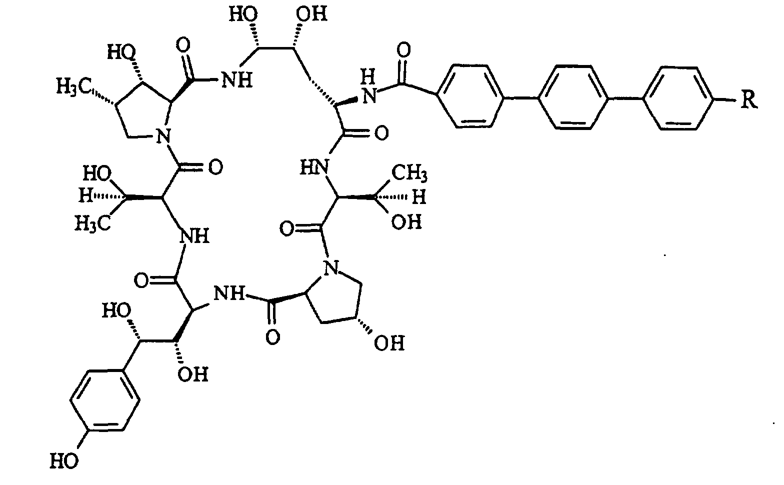 wherein R is -O(CH₂)₃CH₃, -O(CH₂)₄CH₃, -O(CH₂)₅CH₃, -O(CH₂)₂O(CH₂)₃CH₃ or -O(CH₂)₂OC(CH₃)₃.

15. A compound according to claim 14 wherein R is -O(CH₂)₄CH₃.

16. A compound according to any of claims 1-15 for use in inhibiting parasitic activity.

17. A compound according to any one of claims 1-15 for use in inhibiting fungal activity.

18. A compound according to any of claims 1-15 for use in inhibiting the growth of organisms responsible for opportunistic infections in immunosuppressed individuals.

19. A compound according to any of claims 1-15 for use in inhibiting the growth of Pneumocystis carinii.

20. A pharmaceutical formulation comprising a compound according to any of claims 1-15 and a suitable pharmaceutical carrier.

21. A process for the preparation of a compound of the formula (1): 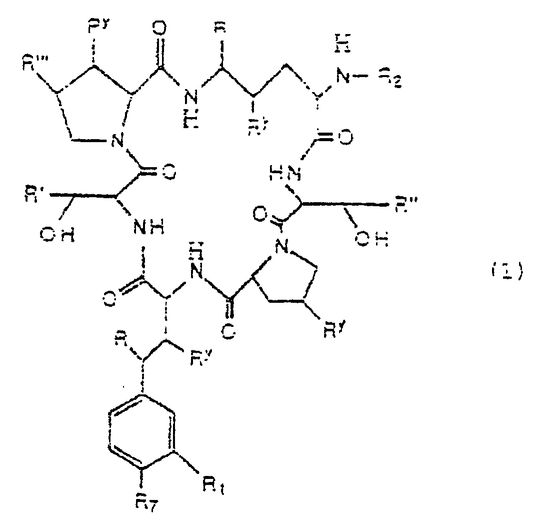 wherein
R' is hydrogen, methyl or NH₂C(O)CH₂-;
R" and R"' is methyl or hydrogen;
R is hydrogen;
R^{y} is hydroxy or hydrogen;
R₁ is hydroxy, or hydrogen;
R₇ is hydroxy, or hydrogen; and
R₂ is hydrogen or acyl;
comprising the step of subjecting a compound of formula (1) wherein R=OH, to a strong acid in the presence of a reducing agent, in a suitable solvent.

22. A process for producing an N-acyl cyclic hexapeptide which process comprises acylating an amino nucleus of Echinocandin B with an active ester of a carboxylic acid represented by the formula 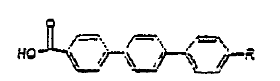 wherein R is -O(CH₂)₃CH₃, -O(CH₂)₄CH₃, -O(CH₂)₅CH₃, -O(CH₂)₂O(CH₂)₃CH₃ or -O(CH₂)₂OC(CH₃)₃.

23. A process for preparing a compound of the formula 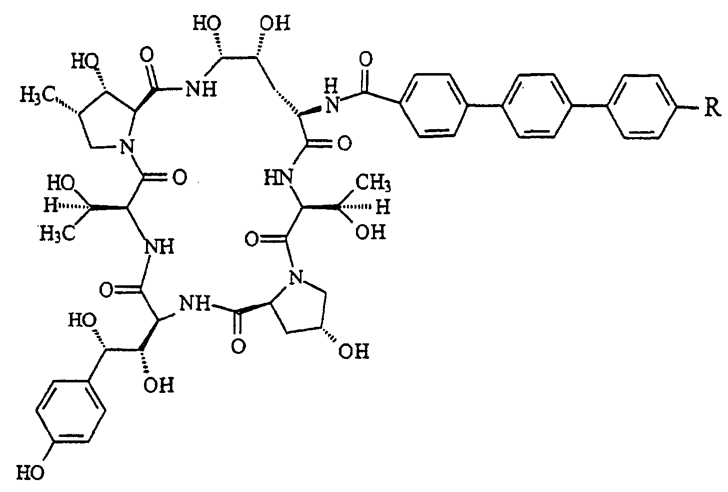 wherein R is -O(CH₂)₃CH₃, -O(CH₂)₄CH₃, -O(CH₂)₅CH₃, -O(CH₂)₂O(CH₂)₃CH₃ or -O(CH₂)₂OC(CH₃)₃ which process comprises acylating an amino nucleus of Echinocandin B with an active ester of a carboxylic acid represented by the formula

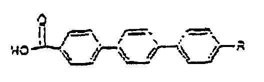

24. A process according to claim 22 or claim 23 wherein R is
-O(CH₂)₄CH₃.

25. A process according to any one of claims 22-24 wherein the active ester is a 2,4,5-trichlorophenyl ester.

26. A process according to any one of claims 22-25 wherein the amine nucleus of Echinocandin B is obtained by N-deacylation of a naturally occurring cyclic hexapeptide.

27. A process according to claims 26 wherein the naturally occurring cyclic hexapeptide is echinocandin B, tetrahydroechinocandin B, mulundocandin, L-671 329, S 31794/FI, sporiofungin or FR 901379.

## Patentansprüche

1. Verbindungen der Formel (1): 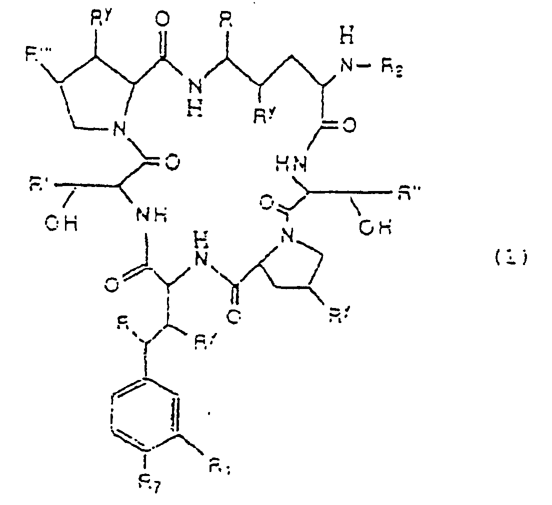 worin
R' Wasserstoff, Methyl oder NH₂C(O)CH₂- ist;
R" und R"' unabhängig voneinander Methyl oder Wasserstoff sind;
R und R^{y} unabhängig voneinander Hydroxy oder Wasserstoff sind;
R₁ Hydroxy, Wasserstoff oder Hydroxysulfonyloxy ist;
R₇ Hydroxy, Wasserstoff, Hydroxysulfonyloxy oder Phosphonooxy ist; und
I) R₂ eine substituierte Benzoylgruppe der Formel 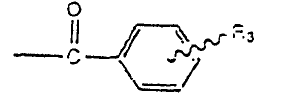 ist, worin R₃ Chinolyl ist; oder
II) R₂ eine Acylgruppe der Formel 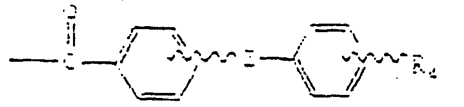 ist, worin
Z -C≡C-, -CH=CH-, oder eine Kohlenstoff-zu-Kohlenstoff-Bindung ist;
(A) R₄ C₃-C₁₂-Cycloalkyl, C₇-C₁₀-Bicycloalkyl, C₇-C₁₄ Tricycloalkyl, C₃-C₁₂-Cycloalkoxy, Naphthyl, Pyridyl, Thienyl, Benzothienyl, Chinolyl oder Phenyl ist; oder
(B) R₄ Phenyl, substituiert durch Amino, C₁-C₁₂-Alkylthio, Halogen, C₁-C₁₂-Alkyl, C₂-C₁₂-Alkenyl, C₂-C₁₂-Alkynyl, substituiertes C₁-C₁₂-Alkyl, substituiertes C₂-C₁₂-Alkenyl, substituiertes C₂-C₁₂-Alkynyl, C₁-C₁₂-Alkoxy, Trifluormethyl, Phenyl, substituiertes Phenyl, Phenyl, substituiert mit einer Polyoxa-Alkylgruppe der Formel
-O-(CH₂)ₘ-[O-(CH₂)ₙ]ₚ-O-(C₁-C₁₂-Alkyl)
worin m und n ganze Zahlen von 2 bis 4 sind und p 0 oder 1 ist; oder
(C) R₄ Phenyl, substituiert mit C₁-C₆-Alkoxy, substituiert durch Fluor, Brom, Chlor oder Iod, ist; oder
(D) R₄ eine Gruppe der Formel
-Y-R₆
ist, worin
Y -C≡C- oder -C=C-, ist; und
R₆ C₁-C₁₂-Alkyl, substituiertes C₁-C₁₂-Alkyl; C₃-C₁₂-Cycloalkyl, C₇-C₁₀-Bicycloalkyl, C₇-C₁₄-Tricycloalkyl, Phenyl, C₃-C₁₂-Cycloalkenyl, Naphthyl, Benzothiazolyl, Thienyl, Phenyl, substituiert durch Amino, C₁-C₁₂-Alkylthio, Halogen, C₁-C₁₂-Alkyl, C₂-C₁₂-Alkenyl, C₂-C₁₂-Alkynyl, C₁-C₁₂-Alkoxy, Trifluormethyl, -O-(CH₂)_{p'}-W-R₅, worin p eine ganze Zahl von 2 bis 4 ist, W Pyrrolidino, Piperidino oder Piperazino ist, ist, und R₅ Wasserstoff, C₁-C₁₂-Alkyl, C₃-C₁₂-Cycloalkyl, Benzyl oder C₃-C₁₂-Cycloalkylmethyl ist; oder C₁-C₆-Alkoxy, substituiert durch Fluor, Brom, Iod oder Chlor, ist; oder
R₆ ein Phenyl, substituiert durch eine Polyoxa-Alkylgruppe der Formel
-O-(CH₂)ₘ-[O-(CH₂)ₙ]ₚ-O-(C₁-C₁₂-Alkyl)
ist, worin m, n und p wie oben definiert sind, oder
R₂ eine Acylgruppe der Formel 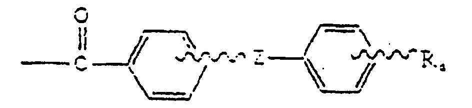 ist, worin
Z -C≡C- oder -CH=CH- ist;
A) R₄ Wasserstoff, C₂-C₁₂-Alkynyl, substituiertes C₂-C₁₂-Alkynyl, C₁-C₁₂-Alkoxy ist; oder
B) R₄ C₁-C₁₂-Alkoxy, substituiert durch C₃-C₁₂-Cycloalkyl, C₇-C₁₀-Bicycloalkyl, C₇-C₁₄-Tricycloalkyl, C₂-C₁₂-Alkynyl, Amino, C₁-C₄-Alkylamino, Di-(C₁-C₄alkyl)amino, C₁-C₁₂-Alkanoylamino, Phenyl, substituiert mit einer Polyoxa-Alkylgruppe der Formel
-O-(CH₂)ₘ-[O-(CH₂)ₙ]ₚ-O-(C₁-C₁₂-Alkyl),
worin m, n und p wie definiert sind, ist; oder
C) R₄ C₁-C₁₂-Alkoxy, substituiert mit einer Gruppe der Formel 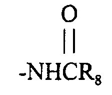 worin R₈ C₁-C₆-Alkoxy, gegebenenfalls substituiert mit Phenyl ist, ist; oder
D) R₄ eine Gruppe der Formel
-O-(CH₂)_{p'}-W-R₅
ist, worin p', W und R₅ wie definiert sind; oder
IV) R₂ eine Gruppe der Formel 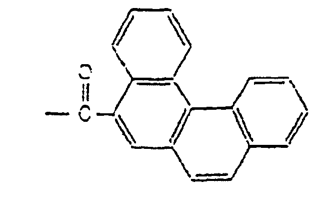 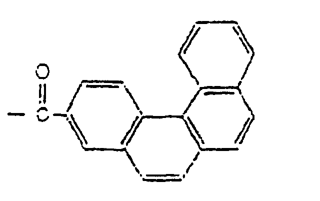 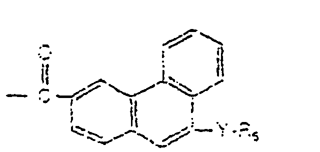 ist, worin Y und R₆ wie oben definiert sind; oder
V) R₂ Naphthoyl ist, substituiert mit R₄, worin
(A) R₄ C₃-C₁₂-Cycloalkyl, C₇-C₁₀-Bicycloalkyl, C₇-C₁₄ Tricycloalkyl, C₃-C₁₂-Cycloalkoxy, Naphthyl, Pyridyl, Thienyl, Benzothienyl, Chinolyl oder Phenyl ist; oder
(B) R₄ Phenyl, substituiert durch Amino, C₁-C₁₂-Alkylthio, Halogen, C₁-C₁₂-Alkyl, C₂-C₁₂-Alkenyl, C₂-C₁₂-Alkynyl, substituiertes C₁-C₁₂-Alkyl, substituiertes C₂-C₁₂-Alkenyl, substituiertes C₂-C₁₂-Alkynyl, C₁-C₁₂-Alkoxy, Trifluormethyl, Phenyl, substituiertes Phenyl, Phenyl, substituiert mit einer Polyoxa-Alkylgruppe der Formel
-O-(CH₂)ₘ-[O-(CH₂)ₙ]ₚ-O-(C₁-C₁₂-Alkyl),
worin m, n und p wie definiert sind, ist; oder
(C) R₄ Phenyl, substituiert mit C₁-C₆-Alkoxy, substituiert durch Fluor, Brom, Chlor oder Iod, ist; oder
(D) R₄ eine Gruppe der Formel
-Y-R₆
ist, worin
Y die gleichen Bedeutungen wie oben definiert besitzt; und
R₆ C₁-C₁₂-Alkyl, substituiertes C₁-C₁₂-Alkyl; C₃-C₁₂-Cycloalkyl, C₇-C₁₀-Bicycloalkyl, C₇-C₁₄-Tricycloalkyl, Phenyl, C₃-C₁₂-Cycloalkenyl, Naphthyl, Benzothiazolyl, Thienyl, Phenyl, substituiert durch Amino, C₁-C₁₂-Alkylthio, Halogen, C₁-C₁₂-Alkyl, C₂-C₁₂-Alkenyl, C₂-C₁₂-Alkynyl, C₁-C₁₂-Alkoxy, Trifluormethyl, -O-(CH₂)_{p'}-W-R₅, oder C₁-C₆-Alkoxy, substituiert durch Fluor, Brom, Iod oder Chlor, ist; oder
R₆ ein Phenyl, substituiert durch eine Polyoxa-Alkylgruppe der Formel
-O-(CH₂)ₘ-[O-(CH₂)ₙ]ₚ-O-(C₁-C₁₂-Alkyl)
ist, worin m, n und p wie oben definiert sind; und pharmazeutisch zulässige nichttoxische Salze davon.

2. Verbindung nach Anspruch 1, worin R₁ Hydroxy oder Wasserstoff ist und R₇ Hydroxy oder Wasserstoff ist.

3. Verbindung gemäß Anspruch 1 oder Anspruch 2, worin R', R" und R"' Methyl sind, R₁ Wasserstoff ist und R₇ und R^{y} OH sind.

4. Verbindung gemäß mindestens einem der Ansprüche 1 bis 3, worin R₂ von folgender Formel ist 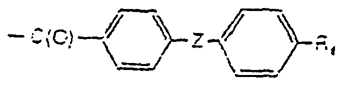 worin
Z eine Kohlenstoff-zu-Kohlenstoff-Bindung ist; und
R₄ C₃-C₇-Cycloalkoxy ist; oder
R₄ Phenyl, substituiert durch C₁-C₁₂-Alkoxy, oder Phenyl, substituiert mit einer Polyoxa-Alkylgruppe der Formel
-O-(CH₂)ₘ-[O-(CH₂)ₙ]ₚ-O-(C₁-C₁₂-Alkyl),
ist; oder
R₄ eine Gruppe der Formel -Y-R₆ ist, worin Y -C≡C- oder -C=C- ist, und R₆ C₁-C₆-Alkyl, Phenyl oder Phenyl, substituiert mit einer Polyoxa-Alkylgruppe der Formel
-O-(CH₂)ₘ-[O-(CH₂)ₙ]ₚ-O-(C₁-C₁₂-Alkyl),
ist.

5. Verbindung gemäß mindestens einem der Ansprüche 1 bis 3, worin R₂ die Formel 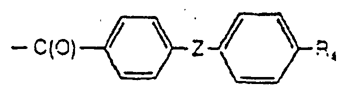 aufweist, worin
Z -C≡C- ist; und
R₄ Phenyl, substituiert durch C₁-C₁₂-Alkoxy- oder Phenyl, substituiert mit einer Polyoxa-Alkylgruppe der Formel
-O-(CH₂)ₘ-[O-(CH₂)ₙ]ₚ-O-(C₁-C₁₂-Alkyl),
ist,
oder R₄ eine Gruppe der Formel
-O-(CH₂)_{p'}-W-R₅
ist,
worin W eine Piperidingruppe ist.

6. Verbindung gemäß mindestens einem der Ansprüche 1 bis 3, worin R Wasserstoff ist.

7. Verbindung gemäß mindestens einem der Ansprüche 1 bis 3, worin R₂ 4-[4-(Phenylethynyl)phenyl]benzoyl oder 4-[4-(n-Butylethynyl)phenyl]benzoyl ist.

8. Verbindung der Formel (1): 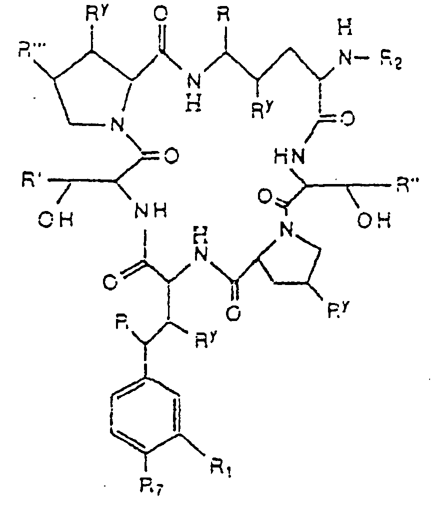 worin
R' Wasserstoff, Methyl oder NH₂C(O)CH₂- ist;
R" und R"' unabhängig voneinander Methyl oder Wasserstoff sind;
R und R^{y} unabhängig voneinander Hydroxy oder Wasserstoff sind;
R₁ Hydroxy, Wasserstoff oder Hydroxysulfonyloxy ist;
R₇ Hydroxy, Wasserstoff, Hydroxysulfonyloxy oder Phosphonooxy ist; und
I) R₂ eine Gruppe der Formel 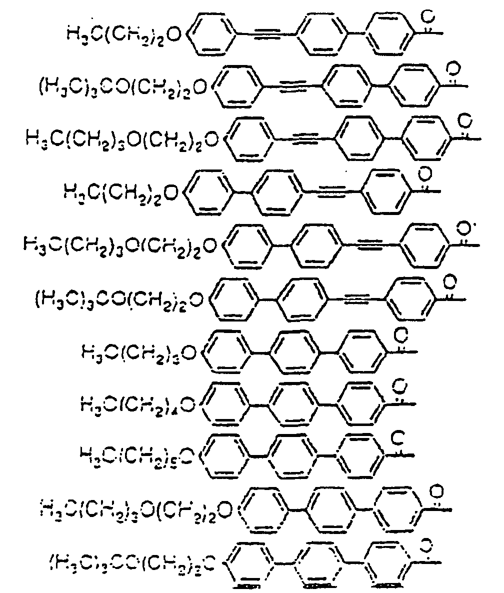 ist, und pharmazeutisch annehmbare Salze davon.

9. Verbindung gemäß Anspruch 8, worin R', R" und R"' Methyl sind, R₁ Wasserstoff ist und R₇ und R^{y} Hydroxy sind.

10. Verbindung der Formel (1): 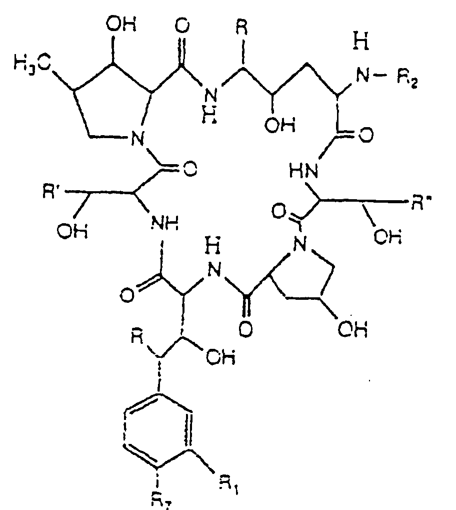 worin
R' Wasserstoff, Methyl oder NH₂C(O)CH₂- ist;
R" Methyl oder Wasserstoff ist;
R Hydroxy oder Wasserstoff ist;
R₁ Hydroxy, Wasserstoff oder Hydroxysulfonyloxy ist;
R₇ Hydroxy, Wasserstoff, Hydroxysulfonyloxy oder Phosphonooxy ist;
R₂ eine Acylgruppe der Formel 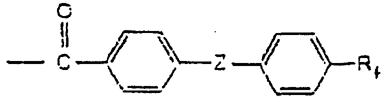 ist, worin Z -C≡C-, -CH=CH- oder eine Kohlenstoff-zu-Kohlenstoff-Bindung ist;
R₄ C₃-C₁₂-Cycloalkyl, C₇-C₁₀-Bicycloalkyl, C₇-C₁₄-Tricycloalkyl, Phenyl, Phenyl, substituiert durch Amino, C₁-C₁₂-Alkylthio, Halogen, C₁-C₁₂-Alkyl, C₁-C₁₂-Alkoxy, Trifluormethyl, Phenyl oder C₁-C₆-Alkoxy, substituiert durch Fluor, Brom, Chlor oder Iod, ist;
oder R₄ C₃-C₁₂-Cycloalkoxy ist;
oder R₄ eine Gruppe der Formel -Y-R₆ ist, worin Y -C≡C- oder -CH=CH- ist, und R₆ C₁-C₁₂-Alkyl, C₁-C₁₂-Alkyl, substituiert durch Phenyl; C₃-C₁₂-Cycloalkyl, Phenyl, C₃-C₁₂-Cycloalkenyl, Naphthyl, Benzthiazol-2-yl oder Phenyl, substituiert durch Amino, C₁-C₁₂-Alkylthio, Halogen, C₁-C₁₂-Alkyl, C₁-C₁₂-Alkenyl, C₁-C₁₂-Alkoxy, Trifluormethyl, -O-(CH₂)_{p'}-W-R₅, worin p' eine ganze Zahl von 2 bis 4 ist; W Pyrrolidino, Piperidino oder Piperazino ist, ist, und R₅ Wasserstoff, C₁-C₁₂-Alkyl, C₃-C₁₂-Cycloalkyl, Benzyl oder C₃-C₁₂-Cycloalkylmethyl ist; oder C₁-C₆-Alkoxy, substituiert durch Fluor, Brom, Iod oder Chlor, ist;
oder R₂ eine Acylgruppe der Formel 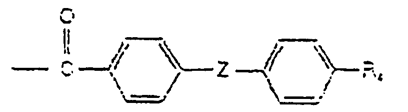 ist, worin Z -C≡C- oder -CH=CH- ist;
R₄ Wasserstoff ist;
R₄ C₁-C₁₂-Alkoxy, C₁-C₁₂-Alkoxy, substituiert durch C₃-C₁₂-Cycloalkyl, C₇-C₁₀-Bicycloalkyl, C₇-C₁₄-Tricycloalkyl, Amino, C₁-C₄-Alkylamino, Di-(C₁-C₄-alkyl)amino, C₁-C₁₂-Alkanoylamino oder eine Gruppe der Formel 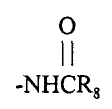 worin R₈ C₁-C₆-Alkoxy, optional substituiert mit Phenyl, ist; oder R₄ eine Gruppe der Formel
-O-(CH₂)_{p'-}W-R₅
ist, oder
R₂ eine Gruppe ist, die aus 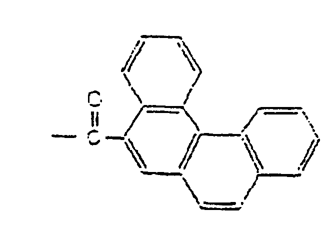 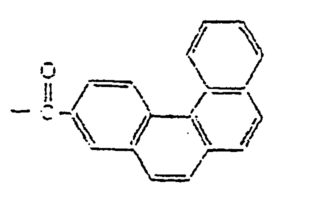 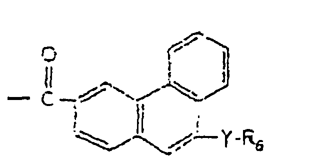 gewählt ist, worin Y und R₆ wie oben definiert sind; oder
R₂ Naphthyl, substituiert mit R₄ ist, worin
R₄ C₃-C₁₂-Cycloalkyl, C₇-C₁₀-Bicycloalkyl, C₇-C₁₄-Tricycloalkyl, Phenyl, Phenyl, substituiert mit Amino, C₁-C₁₂-Alkylthio, Halogen, C₁-C₁₂-Alkyl, C₁-C₁₂-Alkoxy, Trifluormethyl, Phenyl oder C₁-C₆-Alkoxy, substituiert durch Fluor, Brom, Chlor oder Iod, ist;
oder R₄ C₃-C₁₂-Cycloalkoxy ist;
oder R₄ eine Gruppe der Formel -Y-R₆ ist, worin Y die gleiche Bedeutung wie obenstehend besitzt, und R₆
C₁-C₁₂-Alkyl, C₁-C₁₂-Alkyl, substituiert durch Phenyl; C₃-C₁₂-Cycloalkyl, Phenyl, C₃-C₁₂-Cycloalkenyl, Naphthyl, Benzthiazol-2-yl oder Phenyl, substituiert durch Amino, C₁-C₁₂-Alkylthio, Halogen, C₁-C₁₂-Alkyl, C₁-C₁₂-Alkenyl, C₁-C₁₂-Alkoxy, Trifluormethyl, -O-(CH₂)_{p'}-W-R₅ oder C₁-C₆-Alkoxy, substituiert durch Fluor, Brom, Iod oder Chlor, ist; und die pharmazeutisch annehmbaren nicht-toxischen Salze davon.

11. Verbindung gemäß Anspruch 10, worin R₁ nicht Hydroxysulfonyloxy ist und R₇ nicht Hydroxysulfonyloxy oder Phosphonooxy ist.

12. Verbindung der Formel

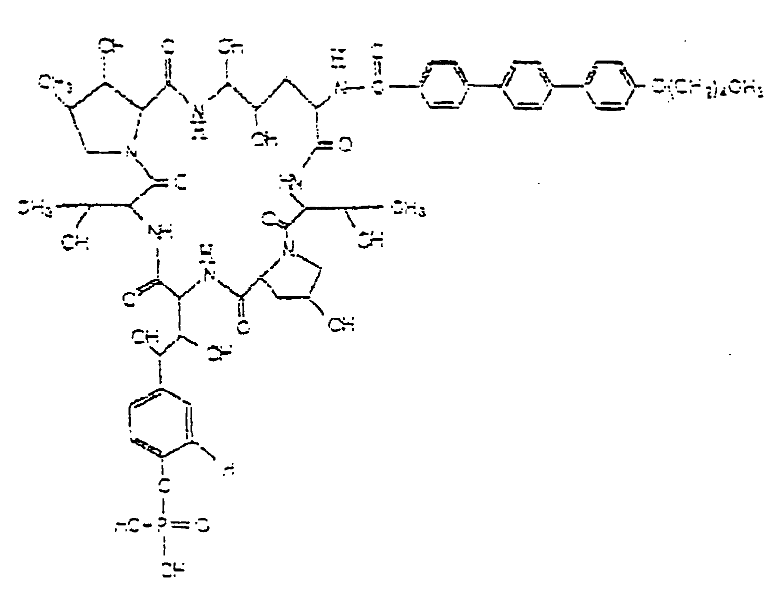

13. Verbindung der Formel

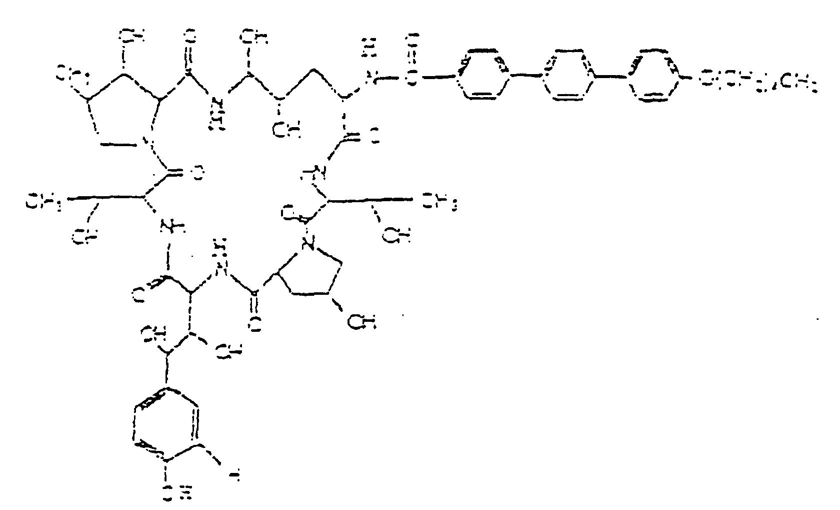

14. Verbindung der Formel 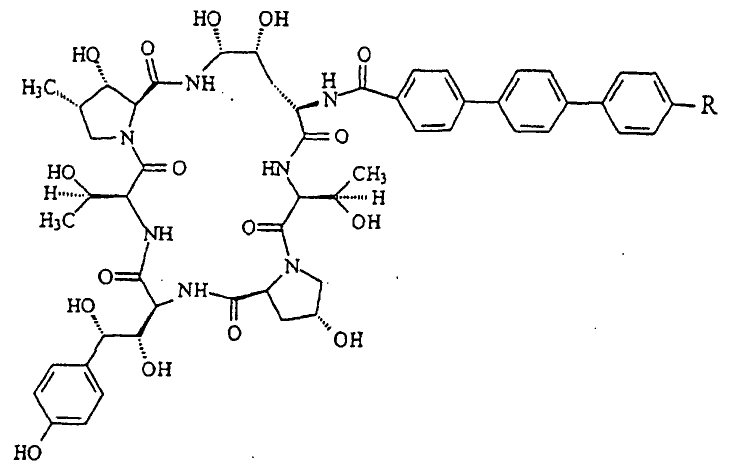 worin R -O(CH₂)₃CH₃, -O(CH₂)₄CH₃, -O(CH₂)₅CH₃, -O(CH₂)₂O(CH₂)₃CH₃ oder -O(CH₂)₂OC(CH₃)₃ ist.

15. Verbindung gemäß Anspruch 14, worin R -O(CH₂)₄CH₃ ist.

16. Verbindung gemäß mindestens einem der Ansprüche 1-15 zur Verwendung bei der Inhibierung parasitischer Aktivität.

17. Verbindung gemäß mindestens einem der Ansprüche 1-15 zur Verwendung bei der Inhibierung fungaler Aktivität.

18. Verbindung gemäß mindestens einem der Ansprüche 1-15 zur Verwendung bei der Inhibierung des Wachstums von Organismen, die für opportunistische Infektionen bei Individuen mit Immunosuppression verantwortlich sind.

19. Verbindung gemäß mindestens einem der Ansprüche 1-15 zur Verwendung bei der Inhibierung des Wachstums von *Pneumocystis carinii*.

20. Pharmazeutische Formulierung, umfassend eine Verbindung gemäß mindestens einem der Ansprüche 1-15 und einen geeigneten pharmazeutischen Träger.

21. Verfahren zur Herstellung einer Verbindung der Formel (1): 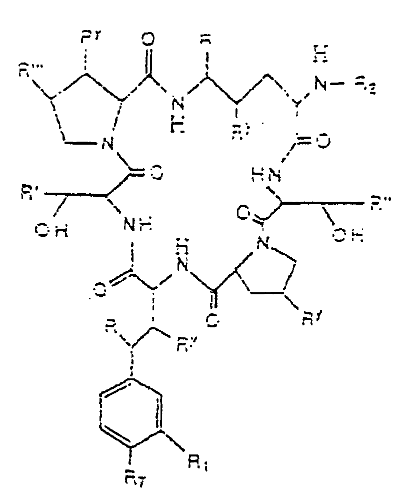 worin
R' Wasserstoff, Methyl oder NH₂C(O)CH₂- ist;
R" und R"' Methyl oder Wasserstoff ist;
R Wasserstoff ist;
R^{y} Hydroxy oder Wasserstoff ist;
R₁ Hydroxy oder Wasserstoff ist;
R₇ Hydroxy oder Wasserstoff ist; und
R₂ Wasserstoff oder Acyl ist;
umfassend die Schritte des Unterziehens einer Verbindung der Formel (1), worin R=OH ist, einer starken Säure in Gegenwart eines Reduktionsmittels in einem geeigneten Lösungsmittel.

22. Verfahren zur Herstellung von cyclischem N-Acyl-Hexapeptid, wobei das Verfahren das Acylieren eines Aminokerns von Echinocandin B mit einem aktiven Ester einer Carbonsäure der Formel 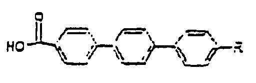 umfasst, worin R -O(CH₂)₃CH₃, -O(CH₂)₄CH₃, -O(CH₂)₅CH₃, -O(CH₂)₂O(CH₂)₃CH₃ oder -O(CH₂)₂OC(CH₃)₃ ist.

23. Verfahren zur Herstellung einer Verbindung der Formel 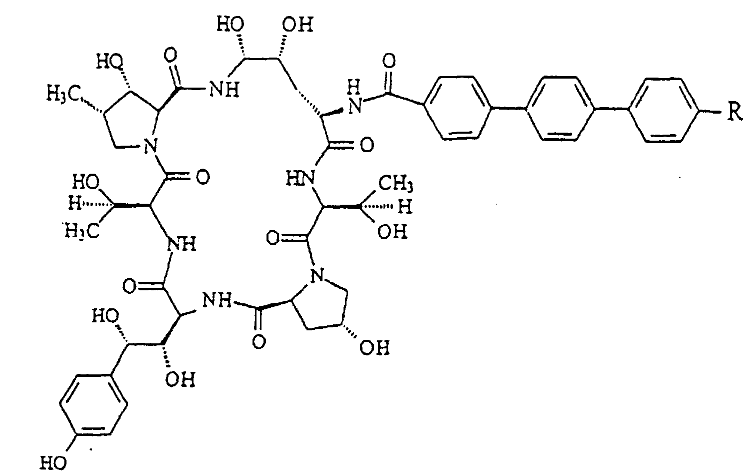 worin R -O(CH₂)₃CH₃, -O(CH₂)₄CH₃, -O(CH₂)₅CH₃, -O(CH₂)₂O(CH₂)₃CH₃ oder -O(CH₂)₂OC(CH₃)₃ ist, wobei das Verfahren die Acylierung eines Aminokerns von Echinocandin B mit einem aktiven Ester einer Carbonsäure der Formel 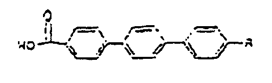 umfasst.

24. Verfahren gemäß Anspruch 22 oder Anspruch 23, worin R -O(CH₂)₄CH₃ ist.

25. Verfahren gemäß mindestens einem der Ansprüche 22-24, bei dem der aktive Ester ein 2,4,5-Trichlorphenylester ist.

26. Verfahren gemäß mindestens einem der Ansprüche 22-25, bei dem der Aminkern von Echinocandin B erhalten wird durch die N-Deacylierung eines natürlich auftretenden cyclischen Hexapeptids.

27. Verfahren gemäß Anspruch 26, bei dem das natürlich auftretende cyclische Hexapeptid Echinocandin B, Tetrahydroechinocandin B, Mulundocandin, L-671 329, S 31794/FI, Sporiofungin oder FR 901379 ist.

## Revendications

1. Composé de formule (1) : 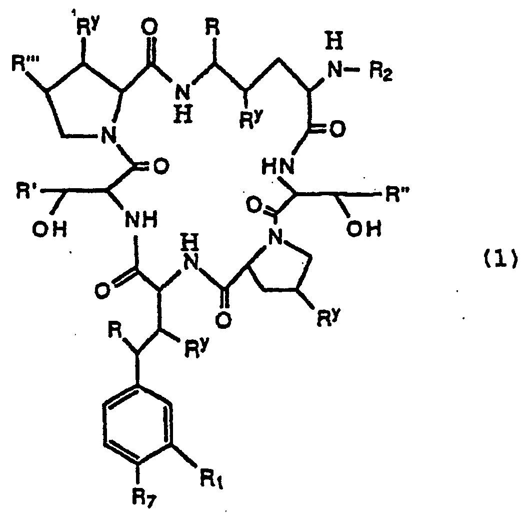 dans laquelle
R' est hydrogène, méthyle, ou NH₂C(O)CH₂- ;
R" et R"' sont indépendamment méthyle ou hydrogène ;
R et R^{y} sont indépendamment hydroxy ou hydrogène ;
R₁ est hydroxy, hydrogène ou hydroxysulfonyloxy ;
R₇ est hydroxy, hydrogène, hydroxysulfonyloxy ou phosphonooxy ; et
I) R₂ est un groupe benzoyle substitué représenté par la formule 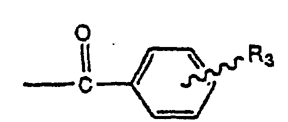 dans laquelle R₃ est quinolyle ; ou
II) R₂ est un groupe acyle représenté par la formule 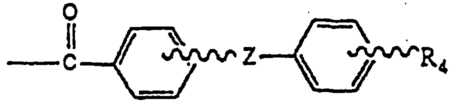 dans laquelle
Z est -C≡C-, -CH=CH- ou une liaison carbone-carbone ;
A) R₄ est un groupe cycloalkyle en C₃ à C₁₂, bicycloalkyle en C₇ à C₁₀, tricycloalkyle en C₇ à C₁₄, cycloalcoxy en C₃ à C₁₂, naphtyle, pyridyle, thiényle, benzothiényle, quinolyle ou phényle ; ou
B) R₄ est un groupe phényle substitué par un groupe amino, alkylthio en C₁ à C₁₂, halogène, alkyle en C₁ à C₁₂, alcényle en C₂ à C₁₂, alcynyle en C₂ à C₁₂, alkyle en C₁ à C₁₂ substitué, alcényle en C₂ à C₁₂ substitué, alcynyle en C₂ à C₁₂ substitué, alcoxy en C₁ à C₁₂, trifluorométhyle, phényle, phényle substitué, phényle substitué par un groupe polyoxa-alkyle représenté par la formule
-O-(CH₂)ₘ-[O-(CH₂)ₙ]ₚ-O-(alkyle en C₁ à C₁₂)
dans laquelle m et n sont des entiers de 2 à 4, et p est 0 ou 1 ; ou
C) R₄ est un groupe phényle substitué par un alcoxy en C₁ à C₆ substitué par fluoro, bromo, chloro ou iodo ; ou
D) R₄ est un groupe représenté par la formule
-Y-R₆
dans laquelle
Y est -C≡C- ou -CH=CH- ; et
R₆ est un alkyle en C₁ à C₁₂, alkyle substitué en C₁ à C₁₂; cycloalkyle en C₃ à C₁₂, bicycloalkyle en C₇ à C₁₀, tricycloalkyle en C₇ à C₁₄, phényle, cycloalcényle en C₃ à C₁₂, naphtyle, benzothiazolyle, thiényle, phényle substitué par amino, alkylthio en C₁ à C₁₂, halogène, alcényle en C₂ à C₁₂, alcynyle en C₂ à C₁₂, alcoxy en C₁ à C₁₂, trifluorométhyle, -O-(CH₂)_{p'}-W-R₅, dans lequel p' est un entier de 2 à 4 ; W est pyrrolidino, pipéridino ou pipérazino, et R₅ est un hydrogène, alkyle en C₁ à C₁₂, cycloalkyle en C₃ à C₁₂, benzyle ou cycloalkylméthyle en C₃ à C₁₂; ou alcoxy en C₁ à C₆ substitué par fluoro, bromo, iodo ou chloro ; ou
R₆ est un phényle substitué par un groupe polyoxa-alkyle représenté par la formule
-O-(CH₂)ₘ-[O-(CH₂)ₙ]ₚ-O-(alkyle en C₁ à C₁₂)
dans laquelle m, n et p sont tels que définis ci-dessus ; ou
III) R₂ est un groupe acyle représenté par la formule 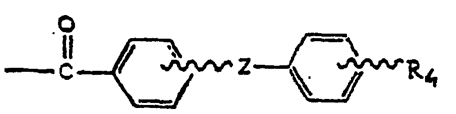 dans laquelle
Z est -C≡C- ou -CH=CH- ;
A) R₄ est un hydrogène, alcynyle en C₂ à C₁₂, alcynyle substitué en C₂ à C₁₂, alcoxy en C₂ à C₁₂; ou
B) R₄ est alcoxy en C₁ à C₁₂ substitué avec cycloalkyle en C₃ à C₁₂, bicycloalkyle en C₇ à C₁₀, tricycloalkyle en C₇ à C₁₄, alcynyle en, amino, alkylamino en C₁ à C_{4,} di-(alkyle en C₁ à C₄)amino, alcanoylamino en C₁ à C₁₂, phényle substitué par un groupe polyoxa-alkyle représenté par la formule
-O-(CH₂)ₘ-[O-(CH₂)ₙ]ₚ-O-(alkyle en C₁ à C₁₂)
dans laquelle m, n et p sont tels que définis ci-dessus ; ou
C) R₄ est un alcoxy substitué avec un groupe de formule 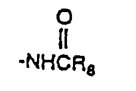 dans laquelle R₈ est un alcoxy en C₁ à C₅ éventuellement substitué par un phényle ; ou
D) R₄ est un groupe représenté par la formule
-O-(CH₂)_{p'}-W-R₅
dans laquelle p', W et .R₅ sont tels que définis ; ou
IV) R₂ est un groupe ayant la formule 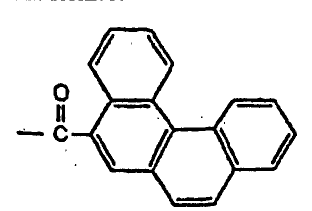 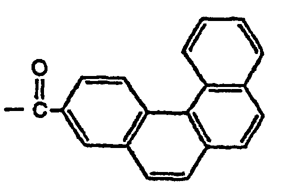 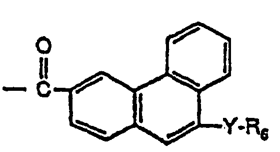 dans laquelle Y et R₆ sont tels que définis ci-dessus ; ou
V) R₂ est un naphtoyle substitué par R₄
dans lequel
A) R₄ est un cycloalkyle en C₃ à C₁₂, bicycloalkyle en C₇ à C₁₀, tricycloalkyle en C₇ à C₁₄, cycloalcoxy en C₃ à C₁₂, naphtyle, pyridyle, thiényle, benzothiényle, quinolyle ou phényle : ou
B) R₄ est un phényle substitué par un amino, alkylthio en C₁ à C₁₂, halogène, alkyle en C₁ à C₁₂, alcényle en C₂ à C₁₂, alcynyle en C₂ à C₁₂, alkyle substitué en C₁ à C₁₂, alcényle substitué en C₂ à C₁₂, alcynyle substitué en C₂ à C₁₂, alcoxy en C₁ à C₁₂, trifluorométhyle, phényle, phényle substitué, phényle substitué avec un groupe polyoxa-alkyle représenté par la formule
-O-(CH₂)ₘ-[O-(CH₂)ₙ]ₚ-O-(alkyle en C₁ à C₁₂)
dans laquelle m, n et p sont tels que définis ; ou
C) R₄ est un phényle substitué par un alcoxy en C₁ à C₆ substitué par un fluoro, bromo, chlore ou iodo ; ou
D) R₄ est un groupe représenté par la formule
dans laquelle
Y a les significations identiques telles que définies ci-dessus ; et
R₆ est un alkyle en C₁ à C₁₂, alkyle substitué en C₁ à C₁₂; cycloalkyle en C₃ à C_{12,} bicycloalkyle en C₇ à C₁₀, tricycloalkyle en C₇ à C₁₄, phényle, cycloalcényle en C₃ à C₁₂, naphtyle, benzothiazolyle, thiényle, phényle substitué par amino, alkylthio en C₁ à C₁₂, halogène, alcényle en C₂ à C₁₂, alcynyle en C₂ à C₁₂, alcoxy en C₁ à C₁₂, trifluorométhyle, -O-(CH₂)_{p'}-W-R₅, ou alcoxy en C₁ à C₆ substitué par fluoro, bromo, iodo ou chloro ; ou
R₆ est un phényle substitué par un groupe polyoxa-alkyle représenté par la formule
-O-(CH₂)ₘ-[O-(CH₂)ₙ]ₚ-O-(alkyle en C₁ à C₁₂)
dans laquelle m, n et p sont tels que définis; et leurs sels non toxiques pharmaceutiquement acceptables.

2. Composé selon la revendication 1 dans lequel R₁ est hydroxy ou hydrogène et R₇ est hydroxy ou hydrogène.

3. Composé selon la revendication 1 ou la revendication 2 dans lequel R', R" et R"' sont un méthyle, R₁ est hydrogène, et R₇ et R^{y} sont OH.

4. Composé selon l'une quelconque des revendications 1 à 3 dans lequel R₂ est de formule 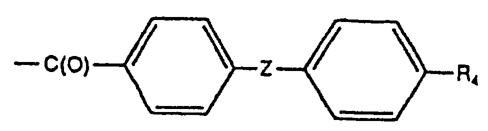 dans laquelle
Z est une liaison carbone-carbone; et
R₄ est un cycloalcoxy en C₃ à C₇; ou
R₄ est un phényle substitué par un alcoxy en C₁ à C₁₂ ou phényle substitué par un groupe polyoxa-alkyle de formule
-O-(CH₂)ₘ-[O-(CH₂)ₙ]ₚ-O-(alkyle en C₁ à C₁₂) ;
ou
R₄ est groupe de formule -Y-R₆, dans laquelle Y est -C≡C- ou -CH=CH- et R₆ est un alkyle en C₁ à C₆, phényle ou phényle substitué par un groupe polyoxa-alkyle de formule
-O-(CH₂)ₘ-[O-(CH₂)ₙ]ₚ-O-(alkyle en C₁ à C₁₂).

5. Composé selon l'une quelconque des revendications 1 à 3 dans lequel R₂ est de formule 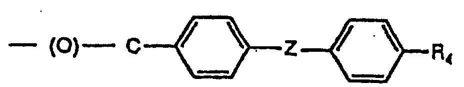 dans laquelle
Z est -C≡C- ; et
R₄ est un phényle substitué par un alcoxy en C₁ à C₁₂ ou phényle substitué par un groupe polyoxa-alkyle de formule
-O-(CH₂)ₘ-[O-(CH₂)ₙ]ₚ-O-(alkyle en C₁ à C₁₂)
ou
R₄ est un groupe de formule
-O-(CH₂)_{p'}-W-R₅
dans laquelle W est un groupe pipéridine.

6. Composé selon l'une quelconque des revendications 1 à 3 dans lequel R est un hydrogène.

7. Composé selon l'une quelconque des revendications 1 à 3 dans lequel R₂ est 4-[4-(phényléthynyl)phényl]benzoyle ou 4-[4-(n-butyléthynyl)phényl]benzoyle.

8. Composé de formule (1) : 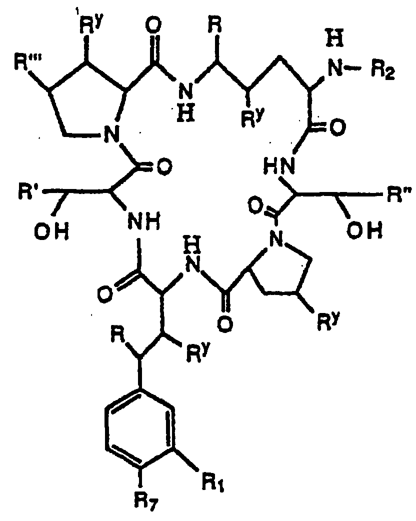 dans laquelle
R' est hydrogène, méthyle, ou NH₂C(O)CH₂- ;
R" et R"' sont indépendamment méthyle ou hydrogène ;
R et R^{y} sont indépendamment hydroxy ou hydrogène ;
R₁ est hydroxy, hydrogène ou hydroxysulfonyloxy ;
R₇ est hydroxy, hydrogène, hydroxysulfonyloxy ou phosphonooxy ; et
I) R₂ est un groupe de formule 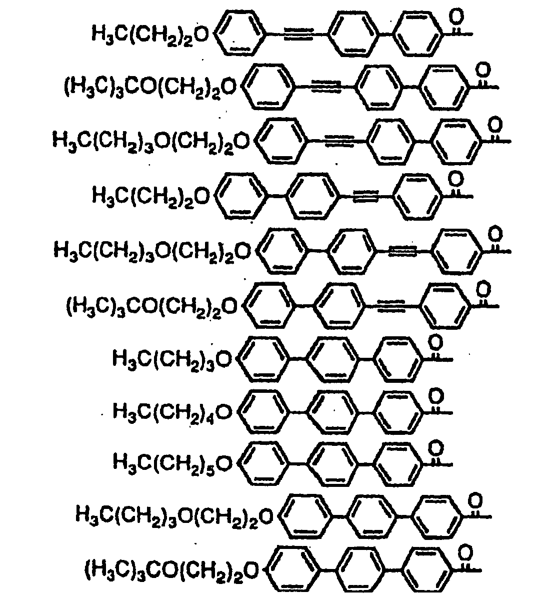 et leurs sels pharmaceutiquement acceptables.

9. Composé selon la revendication 8 dans lequel R', R" et R"' sont un méthyle, R₁ est un hydrogène et R₇ et R^{y} sont hydroxy.

10. Composé de formule (1) : 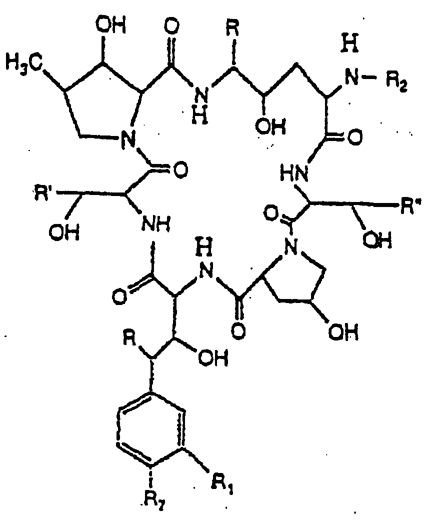 dans laquelle
R' est hydrogène, méthyle, ou NH₂C(O)CH₂- ;
R" est méthyle ou hydrogène ;
R est hydroxy ou hydrogène ;
R₁ est hydroxy, hydrogène ou hydroxysulfonyloxy
R₇ est hydroxy, hydrogène, hydroxysulfonyloxy ou phosphonooxy ;
R₂ est un groupe acyle représenté par la formule 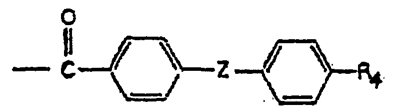 dans laquelle
Z est -C≡C-, -CH=CH- ou une liaison carbone-carbone ;
R₄ est un cycloalkyle en C₃ à C₁₂, bicycloalkyle en C₇ à C₁₀, tricycloalkyle en C₇ à C₁₄, phényle, phényle substitué par amino, alkylthio en C₁ à C₁₂, halogène, alkyle en C₁ à C₁₂, alcoxy en C₁ à C₁₂, trifluorométhyle, phényle ou alcoxy en C₁ à C₆ substitué par fluoro, bromo, chloro ou iodo ;
ou R₄ est un groupe cycloalcoxy en C₃ à C₁₂ ;
ou R₄ est un groupe représenté par la formule -Y-R₆ dans laquelle Y est -C≡C- ou -CH=CH- et R₆ est un alkyle en C₁ à C₁₂, alkyle en C₁ à C₁₂ substitué par phényle; cycloalkyle en C₃ à C₁₂, phényle, cycloalcényle en C₃ à C₁₂, naphtyle, benzothiazol-2-yle ou phényle substitué par amino, alkylthio en C₁ à C₁₂, halogène, alcényle en C₂ à C₁₂, alcoxy en C₁ à C₁₂, trifluorométhyle, -O-(CH₂)_{p'}-W-R₅ dans laquelle p' est un entier de 2 à 4 ; W est pyrrolidino, pipéridino ou pipérazino, et R₅ est un hydrogène, alkyle en C₁ à C₁₂, cycloalkyle en C₃ à C₁₂, benzyle ou cycloalkylméthyle en C₃ à C₁₂ ; ou alcoxy en C₁ à C₆ substitué par fluoro, bromo, iodo ou chloro ;
ou R₂ est un groupe acyle représenté par la formule 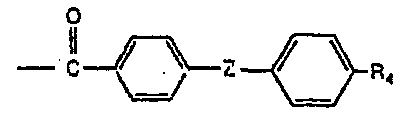
dans laquelle Z est -C≡C- ou -CH=CH- ;
R₄ est un hydrogène ;
R₄ est un alcoxy en C₁ à C₁₂, alcoxy substitué en C₁ à C₁₂ par cycloalkyle en C₃ à C₁₂, bicycloalkyle en C₇ à C₁₀, tricycloalkyle en C₇ à C₁₄, amino, alkylamino en C₁ à C₄, di-(alkyle en C₁ à C₄)amino, alcanoylamino en C₁ à C₁₂ ou un groupe de formule 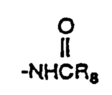 dans laquelle R₈ est un alcoxy en C₁ à C₆ éventuellement substitué par phényle ; ou R₄ est un groupe représenté par la formule
-O-(CH₂)_{p'}-W-R₅
ou
R₂ est un groupe choisi parmi 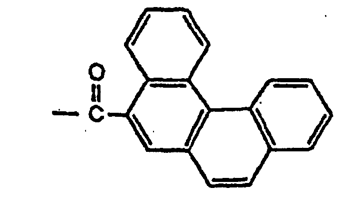 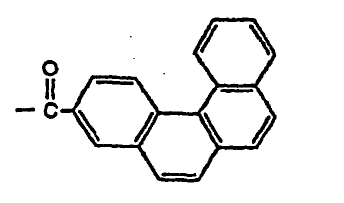 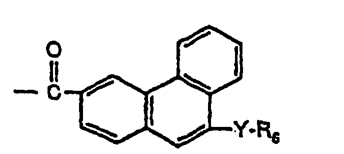 dans laquelle
Y et R₆ sont tels que définis ci-dessus ; ou
R₂ est un naphtoyle substitué par R₄ dans lequel
R₄ est un cycloalkyle en C₃ à C₁₂, bicycloalkyle en C₇ à C₁₀, tricycloalkyle en C₇ à C₁₄, phényle, phényle substitué par amino, alkylthio en C₁ à C₁₂, halogène, alkyle en C₁ à C₁₂, alcoxy en C₁ à C₁₂, trifluorométhyle, phényle ou alcoxy en C₁ à C₆ substitué par fluoro, bromo, chloro ou iodo ;
ou R₄ est un groupe représenté par la formule -Y-R₆ dans laquelle Y a les mêmes significations telles que définies ci-dessus et R₆ est un alkyle en C₁ à C₁₂, alkyle en C₁ à C₁₂ substitué par phényle; cycloalkyle en C₃ à C₁₂, phényle, cycloalcényle en C₃ à C₁₂, naphtyle, benzothiazol-2-yle, ou phényle substitué par amino, alkylthio en C₁ à C₁₂, halogène, alkyle en C₁ à C₁₂, alcényle en C₂ à C₁₂, alcoxy en C₁ à C₁₂, trifluorométhyle, -O-(CH₂)_{p'}-W-R₅, ou alcoxy en C₁ à C₆ substitué par fluoro, bromo, iodo ou chloro ;
et leurs sels non toxiques pharmaceutiquement acceptables.

11. Composé selon la revendication 10 dans lequel R₁ n'est pas un hydroxysulfonyloxy et R₇ n'est ni un hydroxysulfonyloxy ni un phosphonooxy.

12. Composé de formule

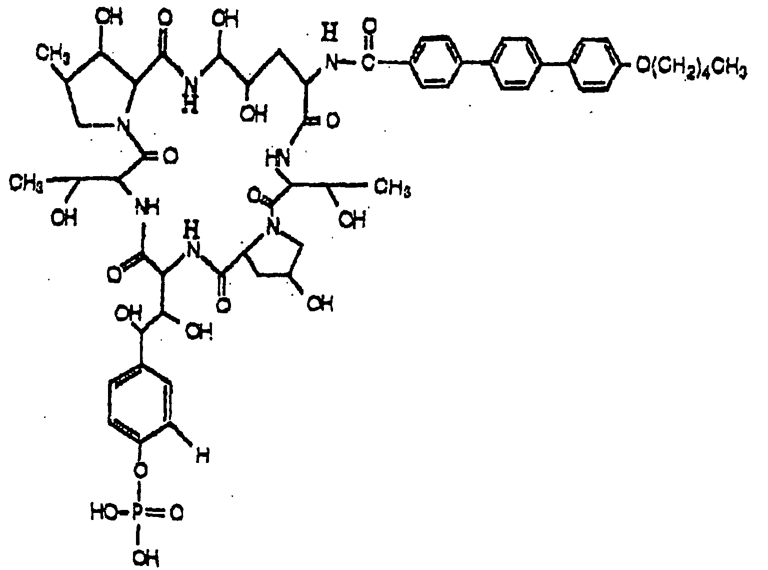

13. Composé de formule

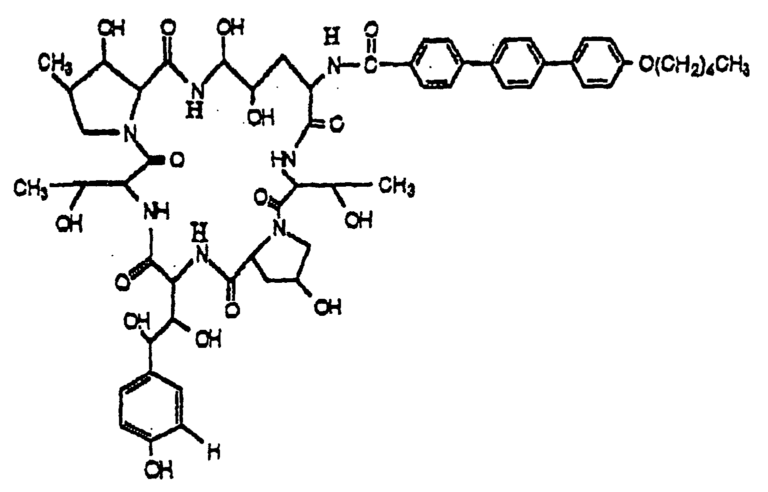

14. Composé de formule 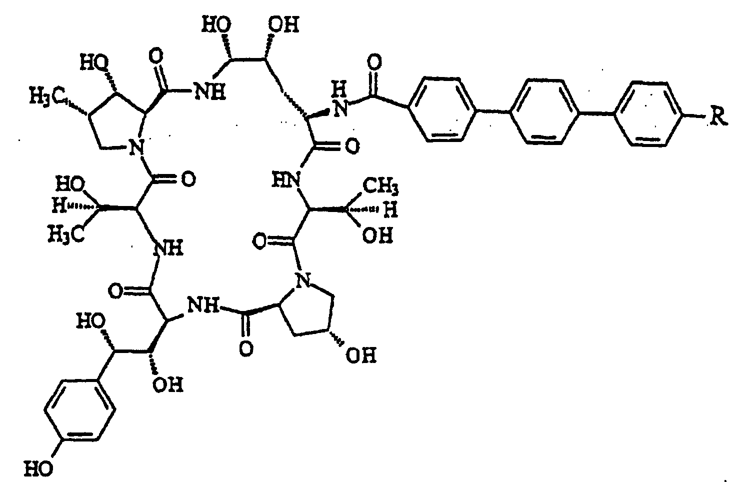 dans laquelle R est -O(CH₂)₃CH₃, -O(CH₂)₄CH₃, -O(CH₂)₅CH₃, -O(CH₂)₂O(CH₂)₃CH₃ ou -O-(CH₂)₂OC(CH₃)₃.

15. Composé selon la revendication 14 dans lequel R est -O(CH₂)₄CH₃.

16. Composé selon l'une quelconque des revendications 1-15 à utiliser pour inhiber une activité parasitique.

17. Composé selon l'une quelconque des revendications 1-15 à utiliser pour inhiber une activité fongique.

18. Composé selon l'une quelconque des revendications 1-15 à utiliser pour inhiber la croissance d'organismes responsables d'infections opportunistes chez les individus immunodéprimés.

19. Composé selon l'une quelconque des revendications 1-15 à utiliser pour inhiber la croissance de Pneumocystis carinii.

20. Formulation pharmaceutique comprenant un composé selon l'une quelconque des revendications 1-15 et un support pharmaceutique approprié.

21. Procédé pour la préparation d'un composé de formule (1) : 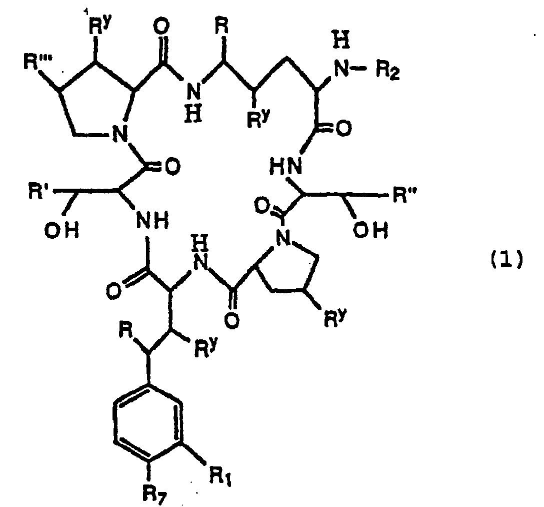 dans laquelle
R' est hydrogène, méthyle, ou NH₂C(O)CH₂- ;
R" et R"' sont méthyle ou hydrogène ;
R est hydrogène ;
R^{y} est hydroxy ou hydrogène ;
R₁ est hydroxy ou hydrogène ;
R₇ est hydroxy ou hydrogène ; et
R₂ est hydrogène ou acyle ;
comprenant l'étape consistant à exposer un composé de formule (1) dans laquelle R = OH, à un acide fort en présence d'un agent réducteur, dans un solvant approprié.

22. Procédé de production d'un N-acylhexapeptide cyclique lequel procédé comprend l'acylation d'un noyau amino d'Echinocandin B avec un ester actif d'un acide carboxylique représenté par la formule 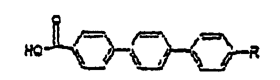 dans laquelle R est -O(CH₂)₃CH₃, -O(CH₂)₄CH₃, -O(CH₂)₅CH₃, -O(CH₂)₂O(CH₂)₃CH₃ ou -O-(CH₂)₂OC(CH₃)₃.

23. Procédé pour la préparation d'un composé de formule 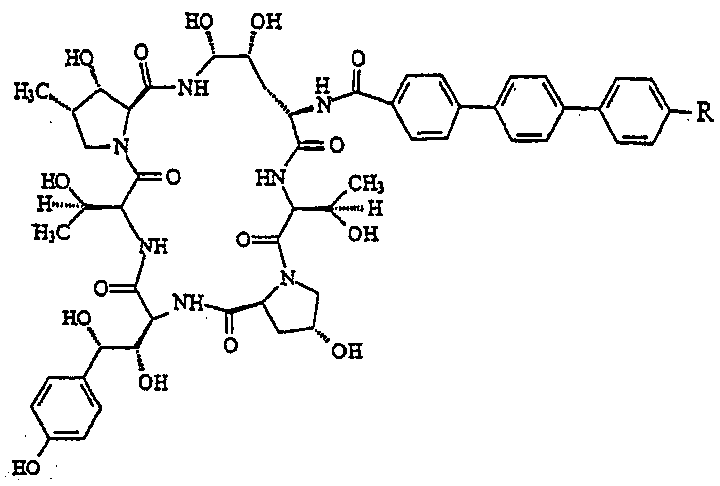 dans laquelle R est -O(CH₂)₃CH₃, -O(CH₂)₄CH₃, -O(CH₂)₅CH₃, -O(CH₂)₂O(CH₂)₃CH₃ ou -O-(CH₂)₂OC(CH₃)₃.lequel procédé comprend l'acylation d'un noyau amino d'Echinocandin B avec un ester actif d'un acide carboxylique représenté par la formule

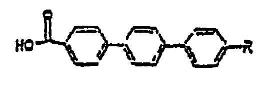

24. Procédé selon la revendication 22 ou la revendication 23 dans lequel R est -O(CH₂)₄CH₃.

25. Procédé selon l'une quelconque des revendications 22-24 dans lequel l'ester actif est un ester de 2,4,5-trichlorophényle.

26. Procédé selon l'une quelconque des revendications 22-25 dans lequel le noyau amine d'Echinocandin B est obtenu par N-désacylation d'un hexapeptide cyclique naturel.

27. Procédé selon la revendication 26 dans lequel l'hexapeptide cyclique naturel est échinocandin B, tétraéchinocandin B, mulundocandin, L-671 329, S 31794/FI, sporiofungin ou FR 901379.
